# EUROPEAN PATENT SPECIFICATION

(11) **EP 3 824 901 B1**
(45) Date of publication and mention of the grant of the patent: **29.04.2026**
(21) Application number: 20201503.8
(22) Date of filing: 13.10.2020
(51) Int. Cl.: A61K 38/16, A61K 38/41, A61P 35/00

(54) **PHARMACEUTICAL COMPOSITION AND USE FOR APPLYING GANODERMA MICROSPORUM IMMUNOMODULATORY PROTEIN AND KEYHOLE LIMPET HEMOCYANIN IN CARCINOMA TREATMENT**
PHARMAZEUTISCHE ZUSAMMENSETZUNG UND VERWENDUNG ZUR ANWENDUNG VON IMMUNMODULATORISCHEM GANODERMA-MICROSPORUM-PROTEIN UND SCHLITZSCHNECKEN-HÄMOCYANIN IN DER KARZINOMBEHANDLUNG
COMPOSITION PHARMACEUTIQUE ET SON UTILISATION POUR L'APPLICATION D'UNE PROTÉINE IMMUNOMODULATOIRE DE GANODERMA MICROSPORUM ET D'UNE HÉMOCYANINE DE PATELLE DANS LE TRAITEMENT DU CARCINOME

(30) Priority: 21.11.2019 US 201962938655 P
(43) Date of publication of application: 26.05.2021
(73) Proprietor: LEE, Feng-Chou, Taipei City 114065 (TW)
(72) Inventor: LEE, Feng-Chou, Taipei City 114065 (TW)
(74) Representative: Zimmermann, Tankred Klaus

(56) References cited:
- US-A1- 2017 080 048
- US-B2- 8 476 238
- US-B2- 9 937 226
- WISHAHI ET AL: "Original Articles: Bladder Cancer: Keyhole-Limpet Hemocyanin Immunotherapy in the Bilharzial Bladder: A New Treatment Modality? Phase II Trial: Superficial Bladder Cancer", JOURNAL OF UROLOGY, LIPPINCOTT WILLIAMS & WILKINS, BALTIMORE, MD, US, vol. 153, no. 3, 1 March 1995 (1995-03-01), pages 926 - 928, XP005576446, ISSN: 0022-5347, DOI: 10.1016/S0022-5347(01)67605-5
- MUSSELLI C ET AL: "Keyhole limpet hemocyanin conjugate vaccines against cancer: the Memorial Sloan Kettering experience", JOURNAL OF CANCER RESEARCH AND CLINICAL ONCOLOGY, SPRINGER INTERNATIONAL, BERLIN, DE, vol. 127, no. SUPPL.2, 1 January 2001 (2001-01-01), pages R20 - R26, XP002259776, ISSN: 0171-5216, DOI: 10.1007/BF01470995
- LIN C H ET AL: "A new immunomodulatory protein from Ganoderma microsporum inhibits epidermal growth factor mediated migration and invasion in A549 lung cancer cells", PROCESS BIOCHEMISTRY, ELSEVIER LTD, GB, vol. 45, no. 9, 1 September 2010 (2010-09-01), pages 1537 - 1542, XP027170824, ISSN: 1359-5113, [retrieved on 20100622]
- DATABASE BIOSIS [online] BIOSCIENCES INFORMATION SERVICE, PHILADELPHIA, PA, US; 1988, JURINCIC C D ET AL: "IMMUNOTHERAPY IN BLADDER CANCER WITH KEYHOLE-LIMPET HEMOCYANIN A RANDOMIZED STUDY", Database accession no. PREV198885124373
- JURINCIC C D ET AL: "IMMUNOTHERAPY IN BLADDER CANCER WITH KEYHOLE-LIMPET HEMOCYANIN A RANDOMIZED STUDY", JOURNAL OF UROLOGY, vol. 139, no. 4, 1988, pages 723 - 726, ISSN: 0022-5347
- DATABASE BIOSIS [online] BIOSCIENCES INFORMATION SERVICE, PHILADELPHIA, PA, US; August 2011 (2011-08-01), HSIN I-LUN ET AL: "GMI, an immunomodulatory protein from Ganoderma microsporum, induces autophagy in non-small cell lung cancer cells", Database accession no. PREV201100539656
- AUTOPHAGY, vol. 7, no. 8, August 2011 (2011-08-01), pages 873 - 882, ISSN: 1554-8627(print), DOI: 10.4161/AUTO.7.8.15698

## Description

### BACKGROUND

### Technology Field

The invention relates to a pharmaceutical composition, in particular for treating a patient with carcinoma, wherein the pharmaceutical composition includes ganoderma microsporum immunomodulatory protein (GMI), phytohemagglutinin (PHA) and keyhole limpet hemocyanin (KLH).

### Description of Related Art

Cancer is a major threat to human health. According to the information published by the Ministry of Health and Welfare of Taiwan, cancer has been the first leading cause of death in Taiwan for 36 consecutive years. This shows that the threat of cancer to human health has reached a level that cannot be ignored. In all types of cancers, more than 80% of cancers belong to carcinoma. The feature of carcinoma is the abnormal mass at the epithelial tissue caused by cell hyperplasia. The proliferation of cancer cells would not be limited like normal cells. The cancer cells would invade other tissues abnormally. Basically, any organ with epithelial tissue in the body may suffer from carcinoma. The most common carcinomas include lung cancer, breast cancer, oral cancer, esophageal cancer, stomach cancer, colon cancer, hepatoma, cervical carcinoma, bladder cancer, pancreatic cancer and cutaneous carcinoma.

The traditional cancer treatments include chemotherapy, surgical treatment, radiation therapy, hormone therapy, biologic therapy, targeted therapy, and therapies which combined the foregoing treatments. However, many types of cancers have resistance to the traditional cancer treatments. In recent years, researches on immunotherapy for the treatment of cancer has been conducted. This method includes administration of the vaccine composition at a site far away from the tumor to induce a systemic tumor-specific active immune response in the patients. Many types of vaccines have been studied, including vaccines containing isolated tumor-associated antigens.

Although many tumor-associated antigens have been identified and many of these antigens have been studied as protein-based or DNA-based vaccines for the treatment or prevention of cancers. However, most of them are still in clinical trials and no therapeutic products have been produced. In addition, one of the challenges in developing cancer vaccines is that tumor-associated antigens are usually derived from the patients themselves. Thus, the cancer vaccines have poor immunogenicity because the immune system is self-regulated and does not recognize its own proteins. Therefore, it is an urgent need to provide a method which can enhance the immunogenicity and the treatment efficacy of cancer vaccine.

Accordingly, it is important to provide a pharmaceutical composition and the use and the method thereof which can induce the specific active immune response of the immune cells against the tumor cells, so as to enhance immunogenicity and treatment efficacy.

### SUMMARY

In view of the foregoing objectives, a purpose of the invention is to provide a pharmaceutical composition and and a pharmaceutical composition for use in treatment of carcinoma, which can induce the specific active immune response of the immune cells against the tumor cells, so as to enhance immunogenicity and treatment efficacy.

To achieve the above objective, the invention provides a pharmaceutical composition comprising a ganoderma microsporum immunomodulatory protein (GMI), a phytohemagglutinin (PHA) and a keyhole limpet hemocyanin (KLH).

To achieve the above objective, the invention also provides a pharmaceutical composition for use in treating carcinoma, wherein the pharmaceutical composition comprises a ganoderma microsporum immunomodulatory protein (GMI), a phytohemagglutinin (PHA) and a keyhole limpet hemocyanin (KLH).

In one embodiment, a concentration of GMI is from 5 µg/ml to 200 µg/ml.

In one embodiment, a concentration of KLH is from 10 µg/ml to 200 µg/ml.

In one embodiment, a weight ratio of GMI to KLH is from 20:1 to 1:40.

In one embodiment, a concentration of PHA is from 1 µg/ml to 20 µg/ml.

In one embodiment, a weight ratio of GMI to PHA is from 200:1 to 1:4.

In one embodiment, a weight ratio of KLH to PHA is from 200:1 to 1:2.

In one embodiment, the pharmaceutical composition is in a dosage form selected from the group consisting of suspension, solution, tablet, capsule, emulsion, dispersion, syrup, granule, transdermal patch, gel, powder, cream, paste, suppository, liquid spray and the combination thereof.

In one embodiment, the carcinoma is lung cancer, breast cancer, oral cancer, esophageal cancer, stomach cancer, colon cancer, hepatoma, cervical carcinoma, bladder cancer, pancreatic cancer, cutaneous carcinoma or the combination thereof.

In one embodiment, the carcinoma is lung cancer, breast cancer, pancreatic cancer, bladder cancer or the combination thereof.

As mentioned above, the efficacy of the pharmaceutical composition and the use and the method thereof of this invention can induce the specific active immune response of the immune cells against the tumor cells, so as to enhance immunogenicity and treatment efficacy.

### BRIEF DESCRIPTION OF THE DRAWINGS

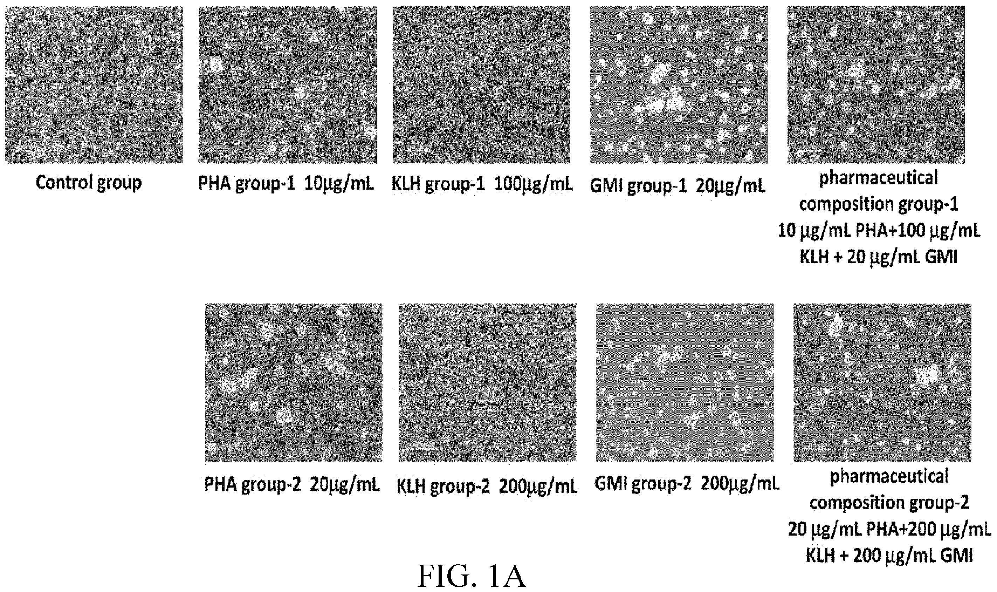

FIG. 1A shows the cell photos of human peripheral blood mononuclear cells (PBMCs) treated by PHA, KLH, GMI, or the pharmaceutical composition of this invention. The photos show the morphology of PBMCs which were observed with the optical microscope.

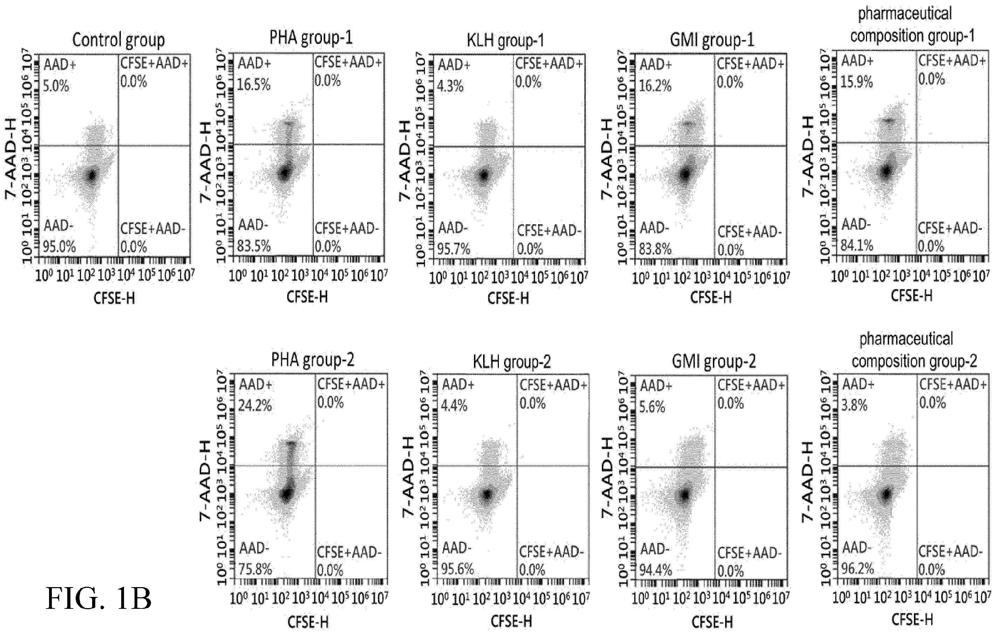

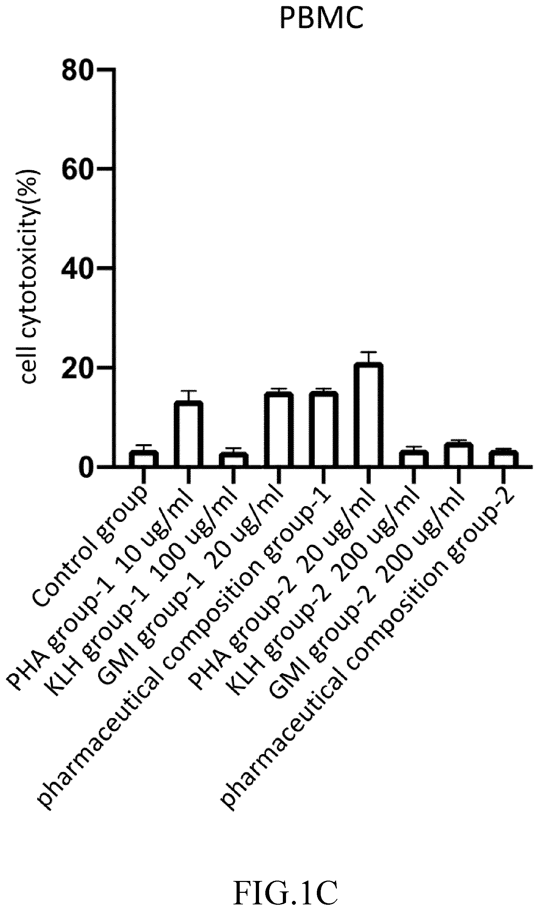

FIGs. 1B-1C show flow cytometer analysis results of PBMCs treated by PHA, KLH, GMI, or the pharmaceutical composition of this invention. FIG. 1B shows the dot plot results of analyzing the fluorescence density of 7-AAD and CFSE in the cells by flow cytometer. The cell cytotoxicity of each treatment could be calculated by these dot plot results. FIG. 1C shows the result of cell cytotoxicity statistics from three independent experiments according to FIG. 1B.

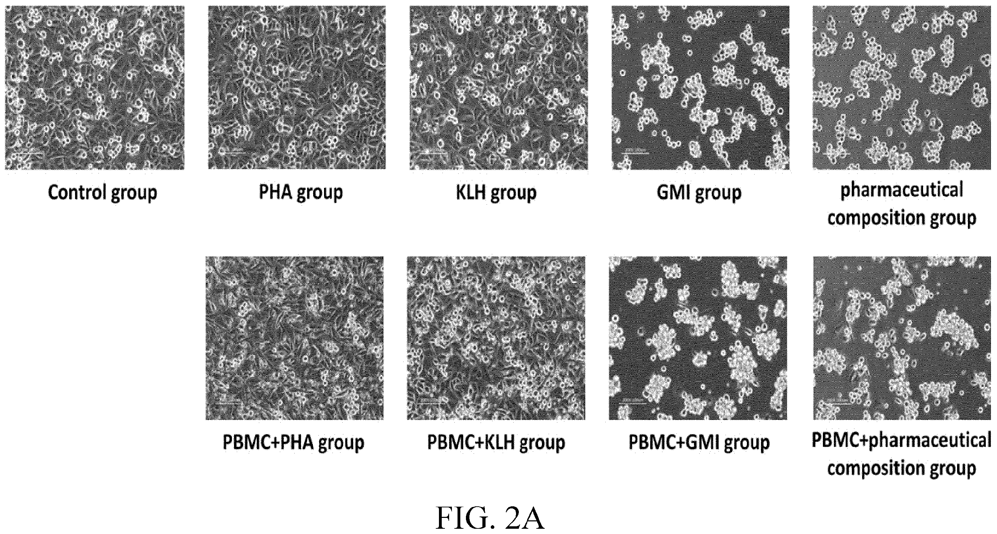

FIGs. 2A-2B show the cell photos of human lung cancer cell line (A549 cells) treated by PHA, KLH, GMI, or the pharmaceutical composition of this invention. The photos show the morphology of A549 cells which were observed with the optical microscope.

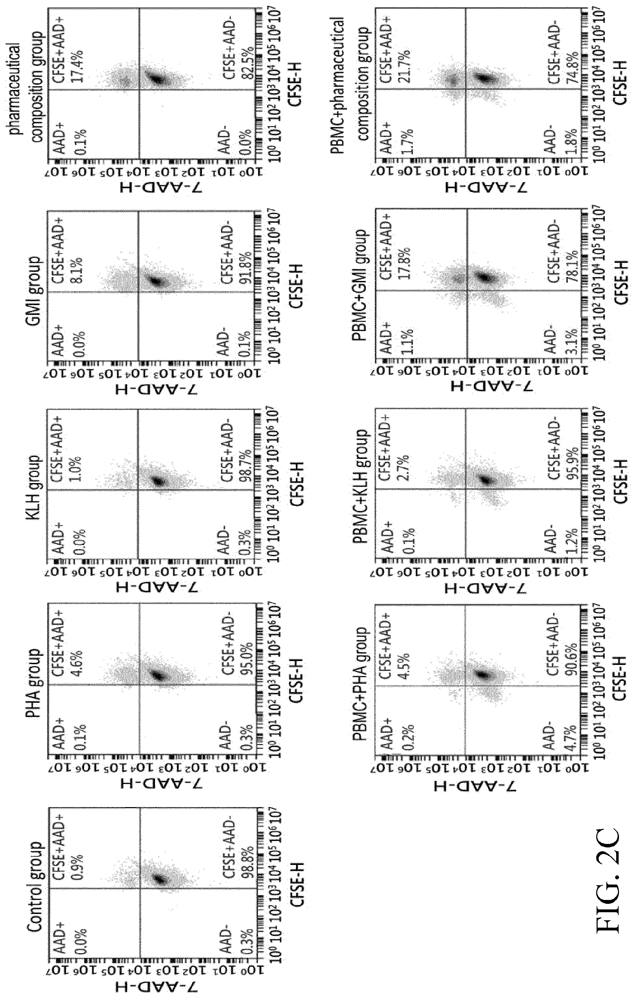

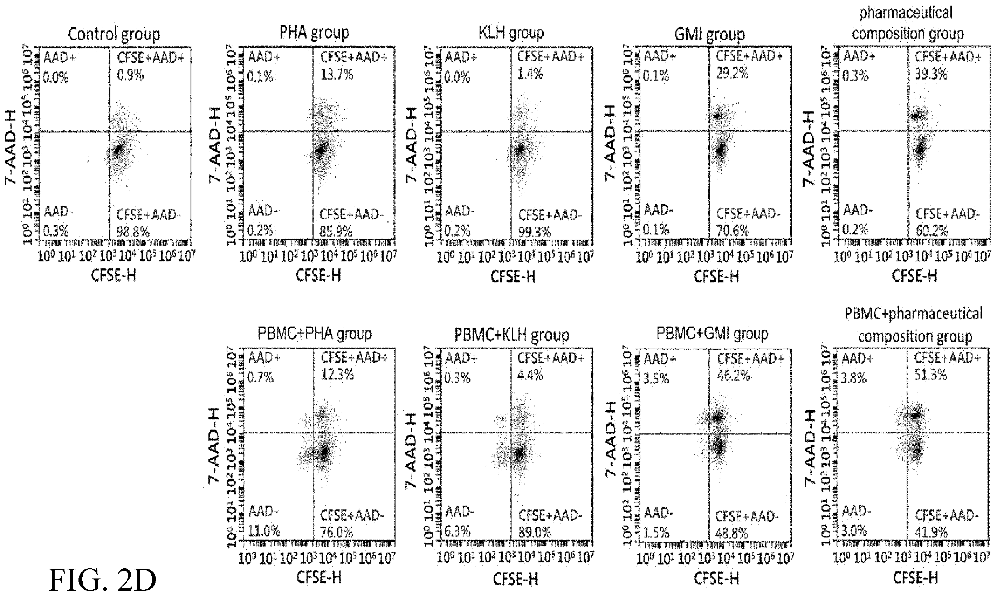

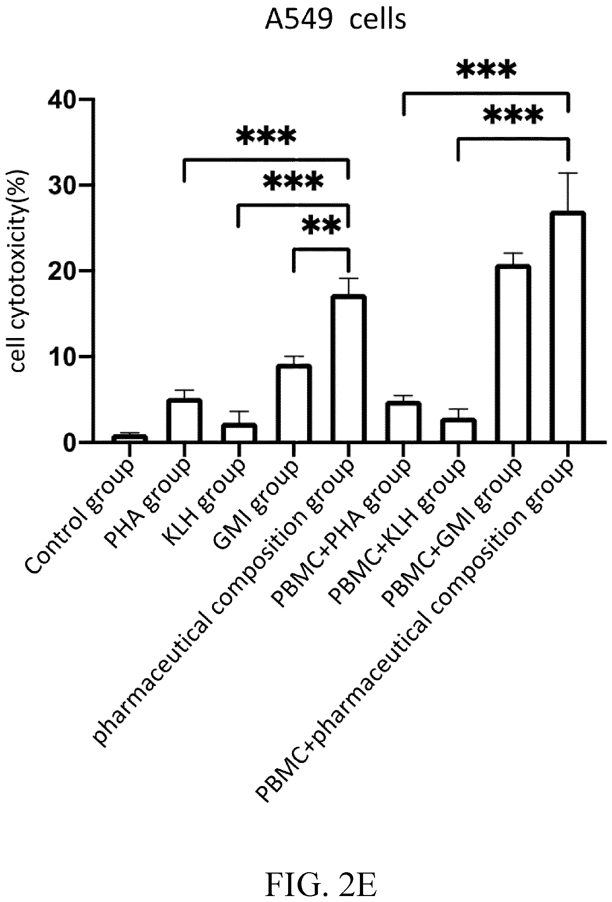

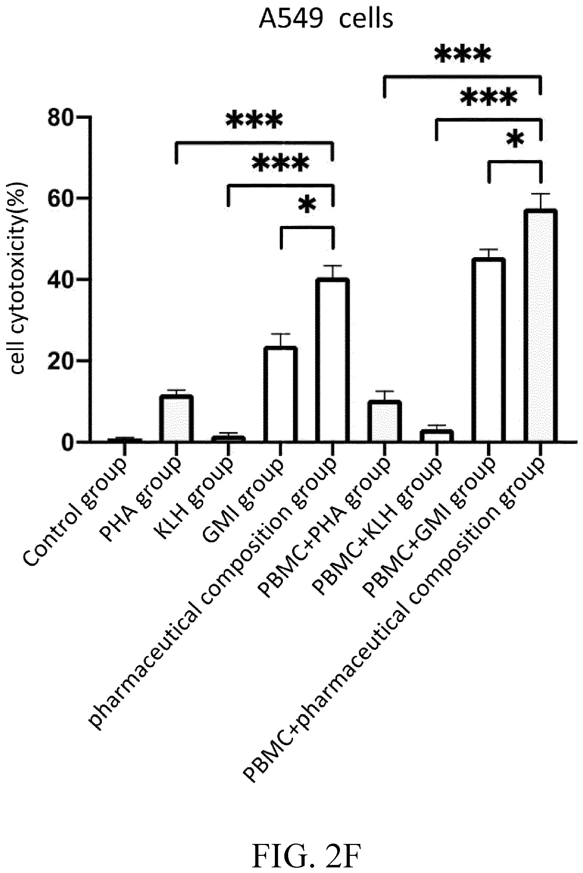

FIGs. 2C-2F show flow cytometer analysis results of A549 cells treated by PHA, KLH, GMI, or the pharmaceutical composition of this invention. FIGs. 2C-2D show the dot plot results of analyzing the fluorescence density of 7-AAD and CFSE in the cells by flow cytometer. The rate of cell cytotoxicity of each treatment could be calculated by these dot plot results. FIG. 2E shows the result of the rates of cell cytotoxicity statistics from three independent experiments according to FIG. 2C. FIG. 2F shows the result of the rates of cell cytotoxicity statistics from three independent experiments according to FIG. 2D.

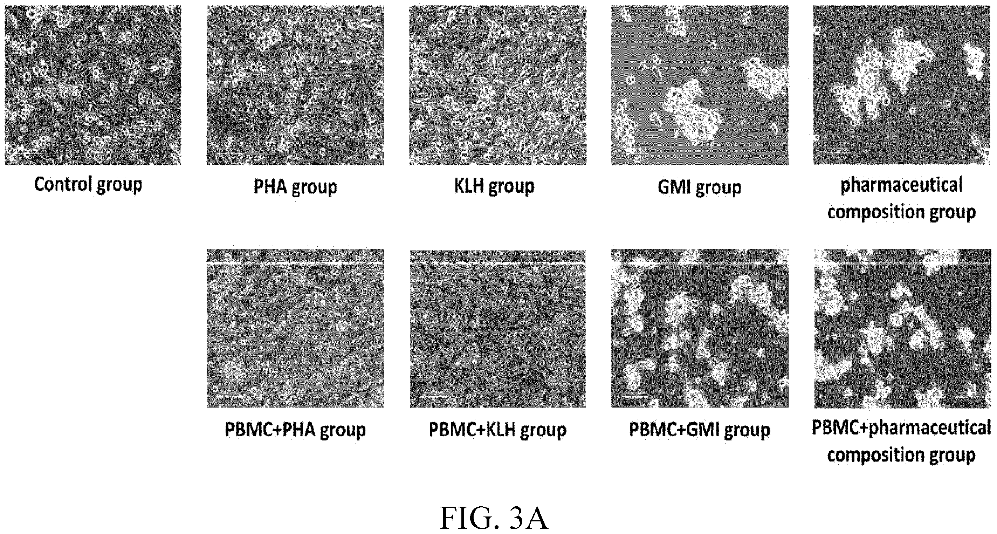

FIGs. 3A-3B show the cell photos of human breast cancer cell line (MDA-MB-231 cells) treated by PHA, KLH, GMI, or the pharmaceutical composition of this invention. The photos show the morphology of MDA-MB-231 cells which were observed with the optical microscope.

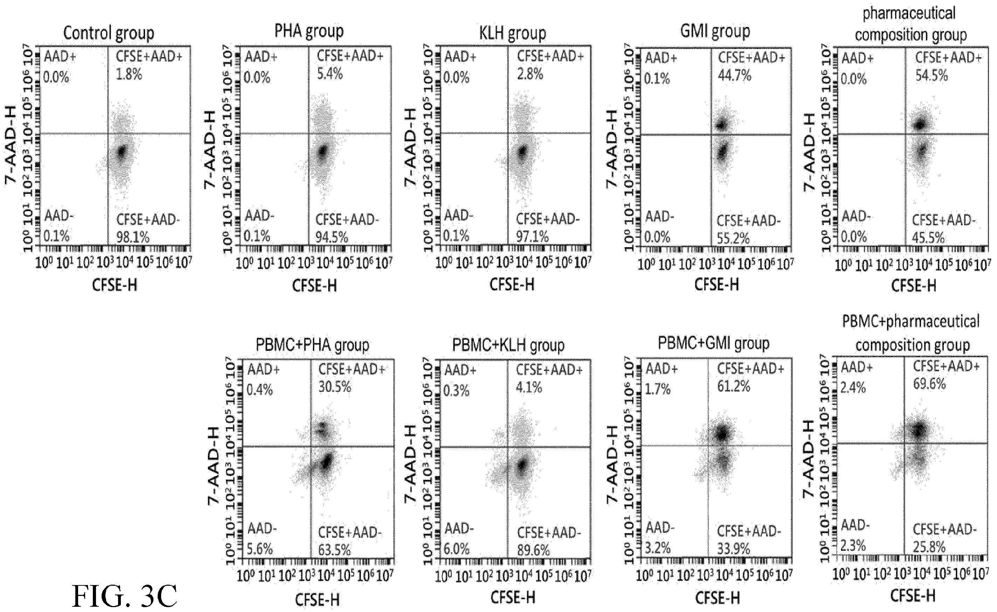

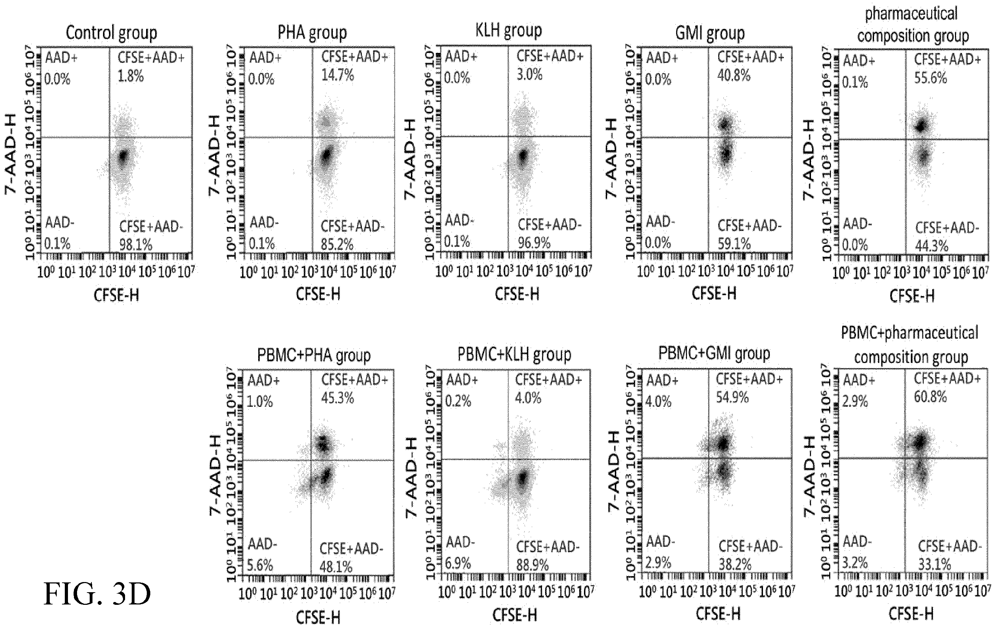

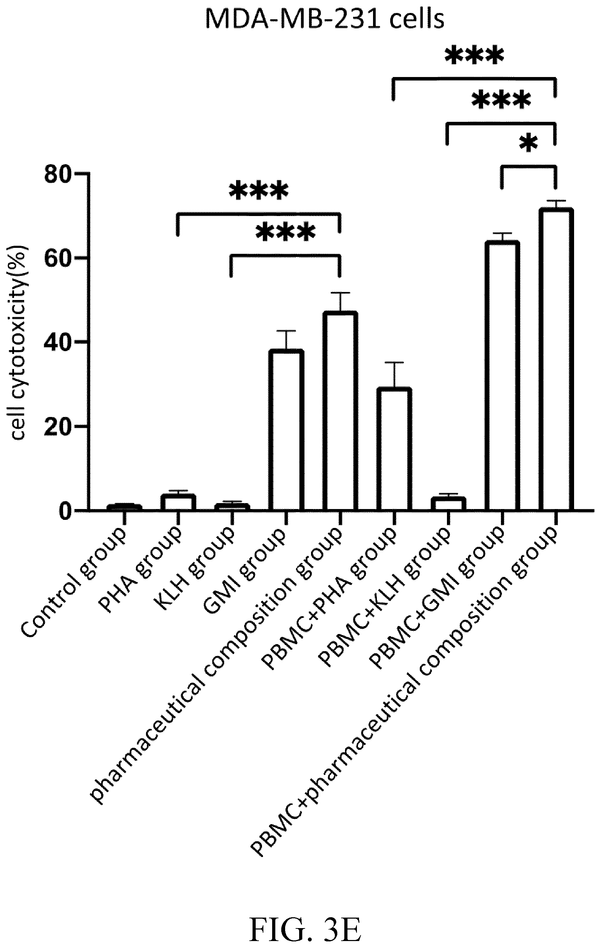

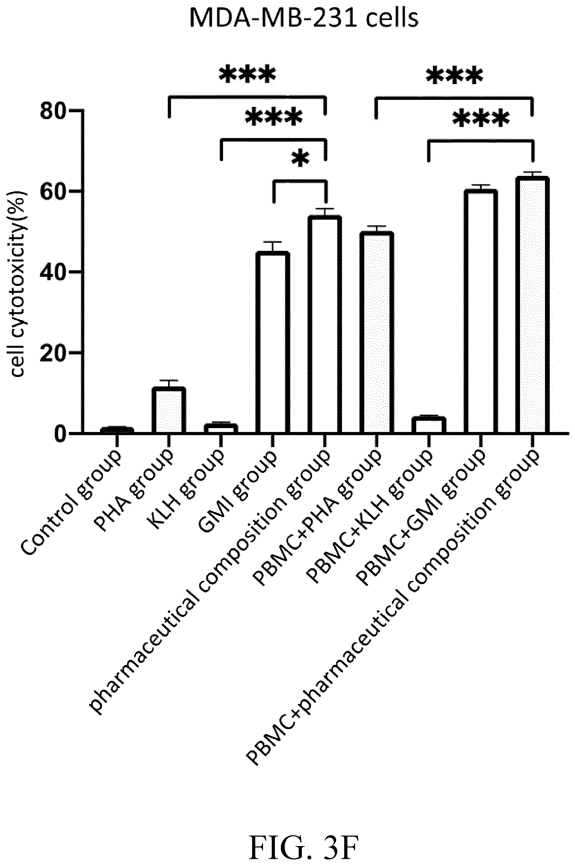

FIGs. 3C-3F show flow cytometer analysis results of MDA-MB-231 cells treated by PHA, KLH, GMI, or the pharmaceutical composition of this invention. FIGs. 3C-3D show the dot plot results of analyzing the fluorescence density of 7-AAD and CFSE in the cells by flow cytometer. The rate of cell cytotoxicity of each treatment could be calculated by these dot plot results. FIG. 3E shows the result of the rates of cell cytotoxicity statistics from three independent experiments according to FIG. 3C. FIG. 3F shows the result of the rates of cell cytotoxicity statistics from three independent experiments according to FIG. 3D.

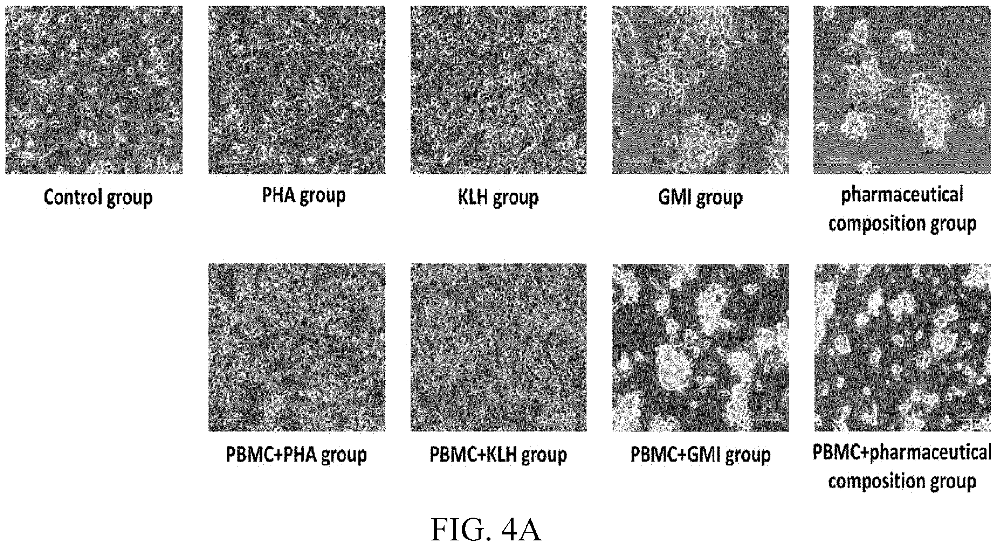

FIGs. 4A-4B show the cell photos of human bladder cancer cell line (T24 cells) treated by PHA, KLH, GMI, or the pharmaceutical composition of this invention. The photos show the morphology of T24 cells which were observed with the optical microscope.

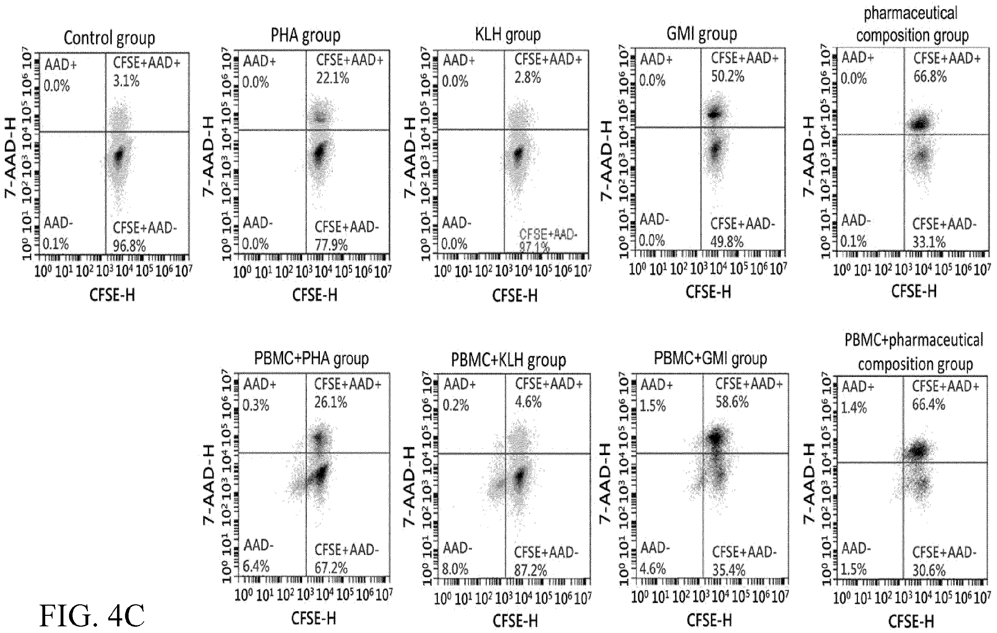

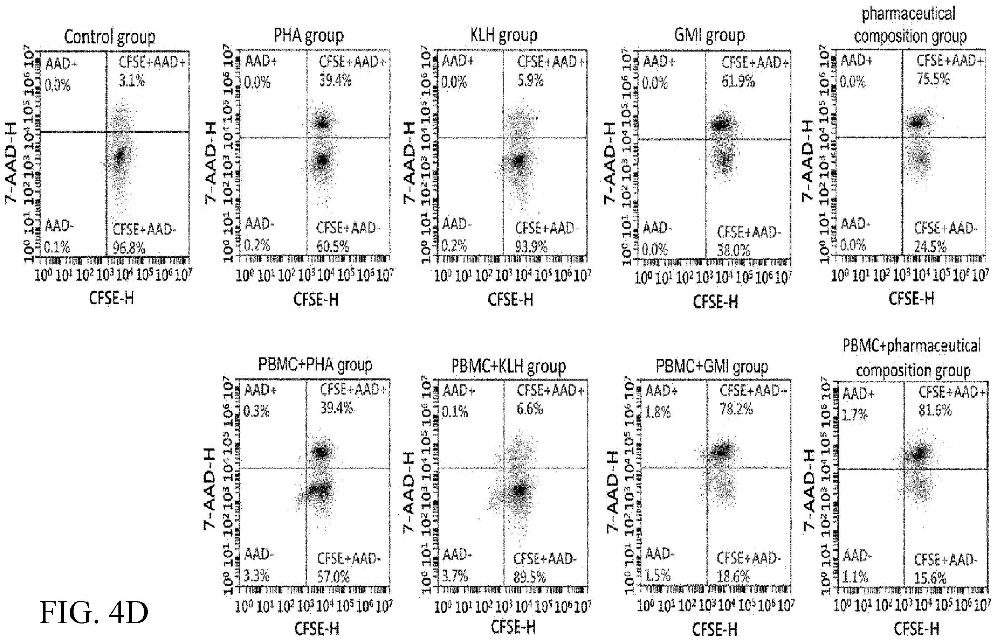

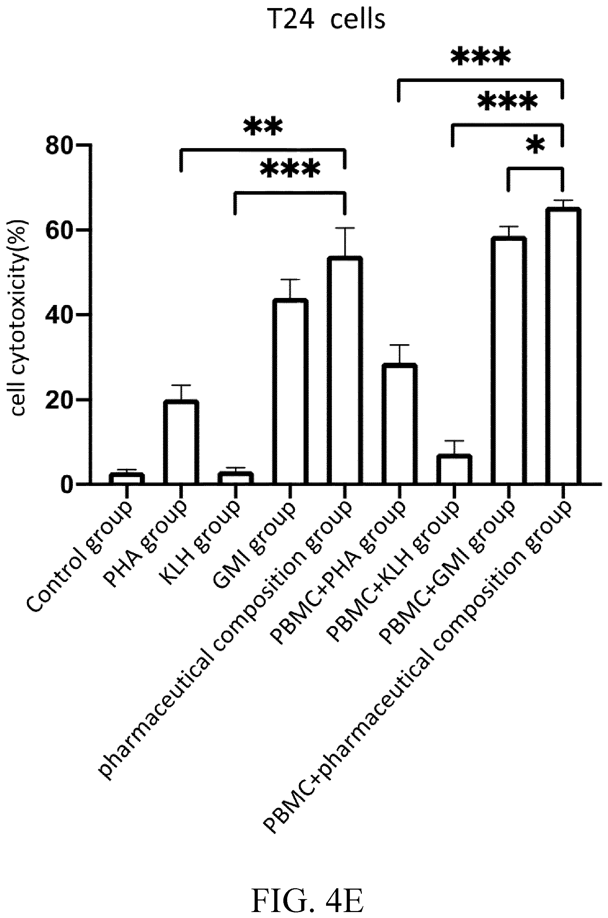

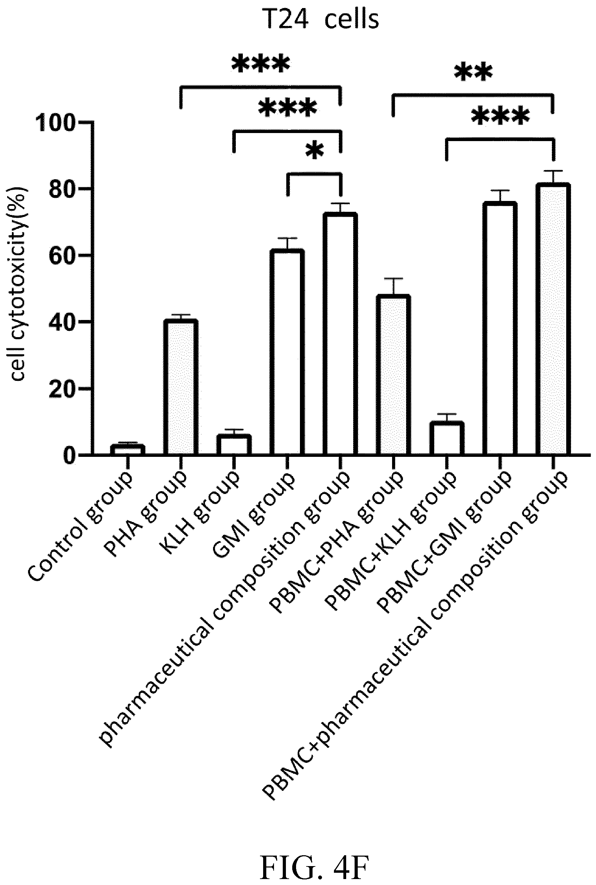

FIGs. 4C-4F show flow cytometer analysis results of T24 cells treated by PHA, KLH, GMI, or the pharmaceutical composition of this invention. FIGs. 4C-4D show the dot plot results of analyzing the fluorescence density of 7-AAD and CFSE in the cells by flow cytometer. The rate of cell cytotoxicity of each treatment could be calculated by these dot plot results. FIG. 4E shows the result of the rates of cell cytotoxicity statistics from three independent experiments according to FIG. 4C. FIG. 4F shows the result of the rates of cell cytotoxicity statistics from three independent experiments according to FIG. 4D.

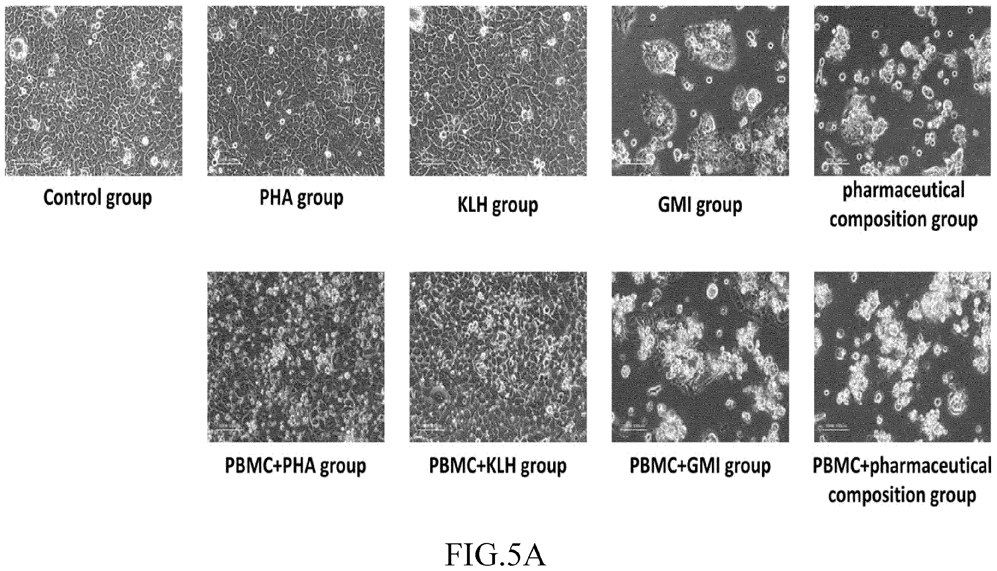

FIGs. 5A-5B show the cell photos of human pancreatic cancer cell line (BxPC-3 cells) treated by PHA, KLH, GMI, or the pharmaceutical composition of this invention. The photos show the morphology of BxPC-3 cells which were observed with the optical microscope.

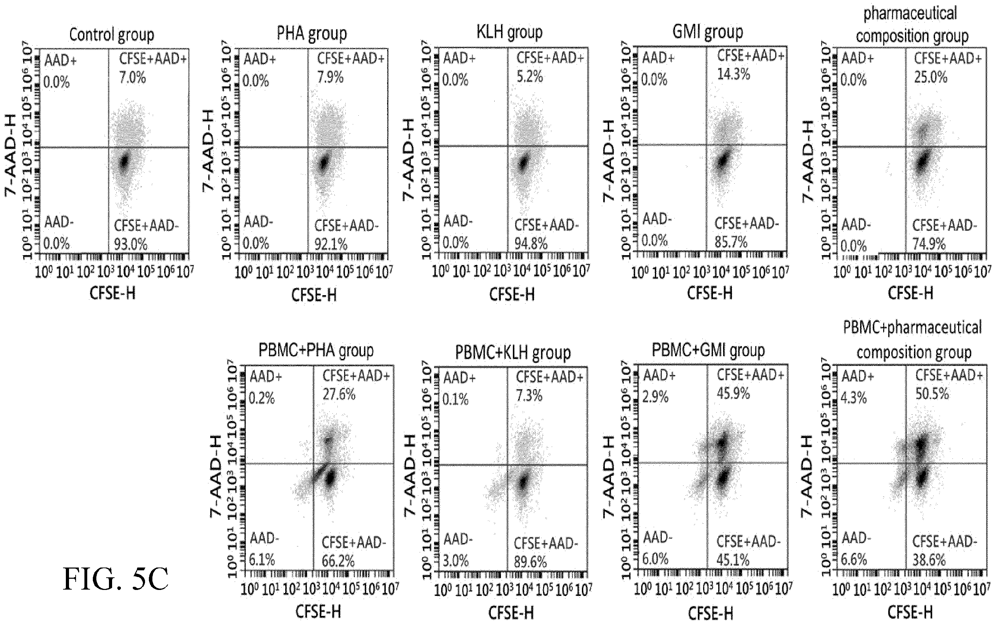

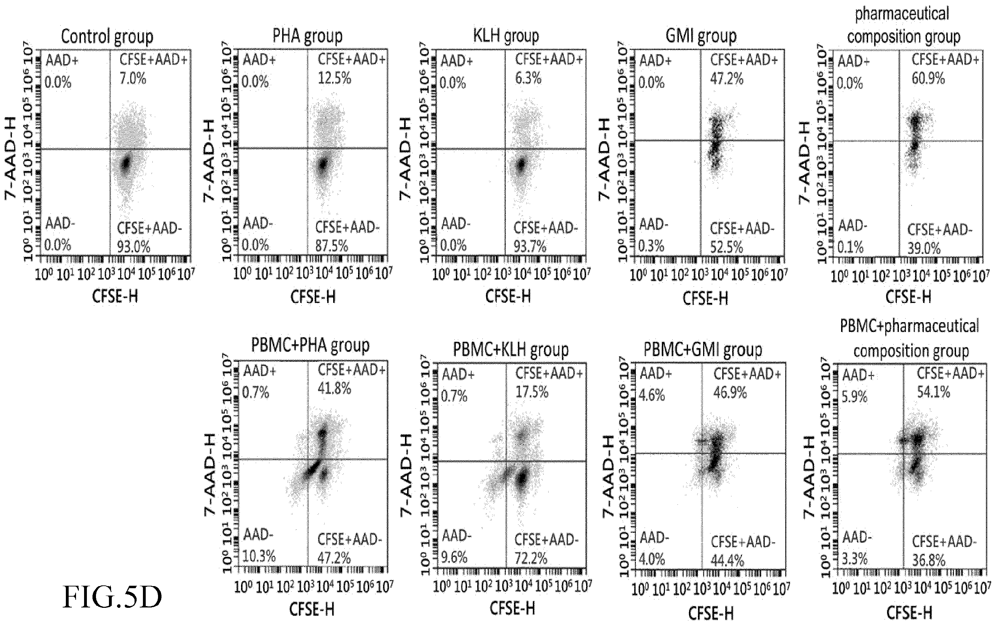

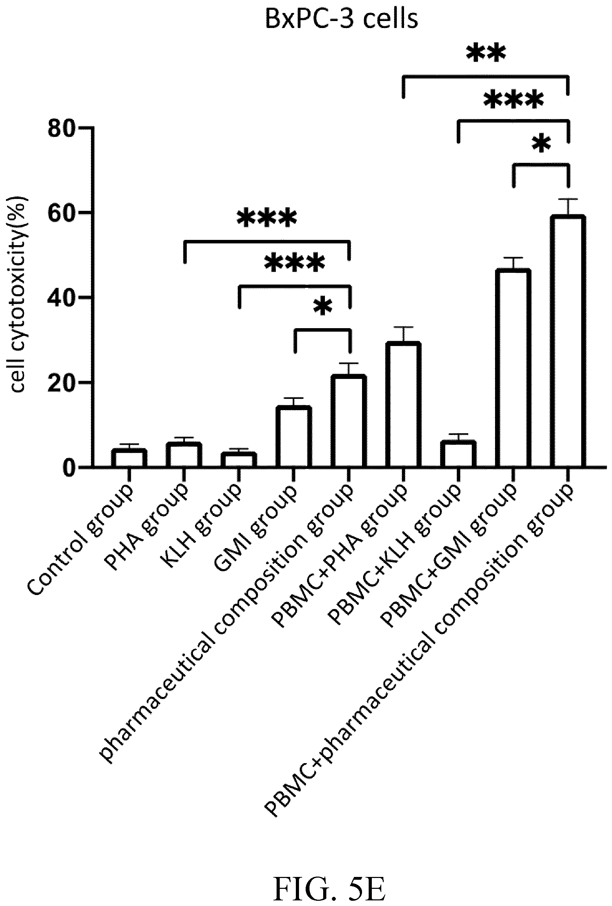

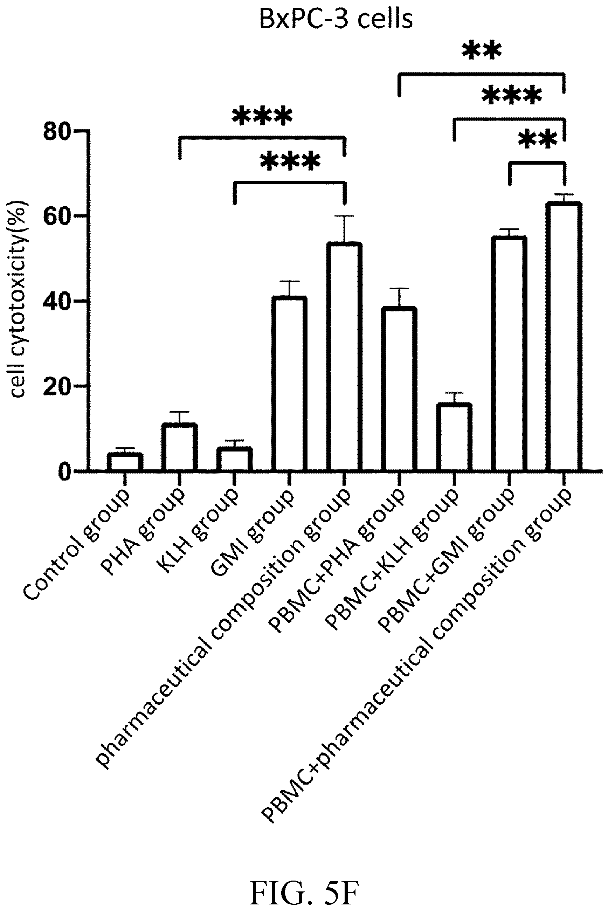

FIGs. 5C-5F show flow cytometer analysis results of BxPC-3 cells treated by PHA, KLH, GMI, or the pharmaceutical composition of this invention. FIGs. 5C-5D show the dot plot results of analyzing the fluorescence density of 7-AAD and CFSE in the cells by flow cytometer. The rate of cell cytotoxicity of each treatment could be calculated by these dot plot results. FIG. 5E shows the result of the rates of cell cytotoxicity statistics from three independent experiments according to FIG. 5C. FIG. 5F shows the result of the rates of cell cytotoxicity statistics from three independent experiments according to FIG. 5D.

### DETAILED DESCRIPTION OF THE DISCLOSURE

The embodiments and examples in this invention will be apparent from the following detailed description, which proceeds with reference to the accompanying figures, wherein the same references relate to the same elements.

The following discussion provides many example embodiments of the inventive subject matter. Although each embodiment represents a single combination of inventive elements, the inventive subject matter is considered to include all possible combinations of the disclosed elements. Thus, if one embodiment comprises elements A, B, and C, and a second embodiment comprises elements B and D, then the inventive subject matter is also considered to include other remaining combinations of A, B, C, or D, even if not explicitly disclosed.

In some embodiments, the numbers expressing quantities of ingredients, properties such as concentration, reaction conditions, and so forth, used to describe and claim certain embodiments of the invention are to be understood as being modified in some instances by the term "about". Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements.

Unless the context dictates the contrary, all ranges set forth herein should be interpreted as being inclusive of their endpoints and open-ended ranges should be interpreted to include only commercially practical values. Similarly, all lists of values should be considered as inclusive of intermediate values unless the context indicates the contrary. The recitation of ranges of values herein is merely intended to serve as a shorthand method of referring individually to each separate value falling within the range. Unless otherwise indicated herein, each individual value is incorporated into the specification as if it were individually recited herein.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims. No language in the specification should be construed as indicating any non-claimed element essential to the practice of the invention.

As used herein, the terms "ganoderma microsporum immunomodulatory protein" and "GMI" refer to the ganoderma microsporum immunomodulatory protein which is discloses in the specification of the published patent which number is TWI474307. GMI is an immunomodulatory protein which is isolated from the ganoderma microsporum or the recombinant protein thereof. This protein has the functions of immunomodulator.

As used herein, the terms "keyhole limpet hemocyanin" and "KLH" refer to a large, multisubunit, oxygen-carrying metalloprotein found in the hemolymph of the giant keyhole limpet (Megathura crenulata). KLH is a heterogeneous glycosylation protein consisted of a subunit which has a molecular weight of around 350,000 to 390,000 Daltons. Each protein is composed by 1 to 20 KLH monomers to form a molecular weight of around 400 kDa (KLH monomer) to 8000 kDa (which is composed of 20 KLH monomers). Each domain of a KLH's subunit contains two copper atoms that together bind to a single oxygen molecule (O₂). When oxygen is bound to hemocyanin, the molecule takes on a distinctive transparent, opalescent blue color. The KLH protein is potently immunogenic but does not cause an adverse immune response in humans. KLH is purified from the hemolymph of Megathura crenulata by a series of steps that typically include ammonium sulfate precipitation and dialysis, and may involve chromatographic purification to obtain the highest purity. In some embodiment, KLH purification may also include endotoxin removal, but this step is often unnecessary because the endotoxin serves as an adjuvant when injected for antibody production. In some embodiment, KLH is a giant multimer (which is composed of 10 or 20 monomers) which has a molecular weight of around 4000 to 8000 kDa. In some embodiment, KLH is a multimer which has the molecular weight of around 8 to 10 million Daltons and 92-107S sedimentation coefficient.

As used herein, the terms "phytohemagglutinin" or "PHA" refer to a non-enzyme protein from a non-immune source isolated from the plants. This protein can agglutinate cells and precipitate monosaccharide or polysaccharide complex. Due to the ability of PHA which can specifically combine to monosaccharide or polysaccharide complex, PHA plays an important role in many signal transduction processes, such as signal transduction, immune response and plant defense. At the same time, PHA has a variety of abilities such as cell agglutination, antiviral, antifungal, and inducing apoptosis or autophagy.

As used herein, the term "pharmaceutical composition" refers to a mixture of GMI, KLH of this invention with/without other components, such as pharmaceutically acceptable carrier, diluent, stabilizer, suspending agent, dispersing agent, thickening agent, and/or excipient. The pharmaceutical composition facilitates administration of the GMI and KLH to a subject.

Pharmaceutical compositions useful in the methods of the invention may be suitable for nasal, inhalational, parenteral, rectal, vaginal, oral, pleural, intrathecal, peritoneal, topical, transdermal, intranasal, buccal, ophthalmic, pulmonary, epidural, intravenous or another route of administration. The useful compositions of this invention can administrate to mammals directly. Other contemplated formulations include microspheres, immunologically-based formulations, liposomal preparations, polymer conjugates, coated particles, and projected nanoparticles.

The routes of administration will be readily apparent to the skilled person and will depend upon any number of factors including the type and severity of the disease being treated, the type and age of the veterinary or human patient being treated, and the like.

The formulations of the pharmaceutical compositions described herein may be prepared by any method known or hereafter developed in the art of pharmacology and pharmaceutics. In general, such preparation methods include the step of bringing the active ingredient into association with a carrier or one or more other accessory ingredients, and then, if necessary or desirable, shaping or packaging the product into the desired single-dose or multi-dose unit.

As used herein, a "unit dose" is a discrete amount of the pharmaceutical composition comprising a predetermined amount of the active ingredient. The amount of the active ingredient is generally equal to the dosage of the active ingredient that would be administered to a subject or a convenient fraction of such a dosage such as, for example, one-half or one-fourth of such a dosage. The unit dosage form may be for a single daily dose or one of multiple daily doses (e.g., about 1 to 3 or more times per day). When multiple daily doses are used, the unit dosage form may be the same or different for each dose.

As used herein, the term "pharmaceutically acceptable" refers to a material, such as a diluent or carrier, which does not abrogate the biological activity or properties of the pharmaceutical composition of this invention, and is relatively non-toxic, i.e., the material may be administered to a subject without causing undesirable biological effects or interacting in a deleterious manner with any of the components of the pharmaceutical composition in which it is contained.

As used herein, the term "pharmaceutically acceptable carrier" includes salts, composition, material or carrier, such as diluent, filler, encapsulating material or excipient, involved in carrying or transporting the pharmaceutical composition of the invention within or to the subject such that the pharmaceutical composition may perform its intended function. Typically, the pharmaceutical composition is carried or transported from one organ or portion of the body to another organ or portion of the body. Each salt or carrier must be compatible with the other ingredients of the formulation, including the pharmaceutical composition of the invention useful within the invention, and not injurious to the subject. Some examples of materials that may serve as pharmaceutically acceptable carriers include: pyrogen-free water; sugars, such as starches, such as corn starch and potato starch; lactose, glucose and sucrose; cellulose, and its derivatives, such as sodium carboxymethyl cellulose, powdered tragacanth; malt; gelatin; talc; excipients, such as cocoa butter and suppository waxes; ethyl cellulose and cellulose acetate; oils, such as peanut oil, cottonseed oil, safflower oil, sesame oil, olive oil, corn oil and soybean oil; glycols, such as propylene glycol; esters, such as ethyl oleate and ethyl laurate; agar; phosphate buffer solutions; polyols, such as glycerin, sorbitol, mannitol and polyethylene glycol; buffering agents, such as magnesium hydroxide and aluminum hydroxide; surface active agents; alginic acid; isotonic saline; Ringer's solution; ethyl alcohol; and other non-toxic compatible substances employed in pharmaceutical formulations.

In certain embodiments, the pharmaceutical compositions of this invention may be formulated by using one or more pharmaceutically acceptable carriers or excipients. In certain embodiments, the pharmaceutical compositions of this invention comprise a therapeutically effective amount of GMI, KLH, and/or PHA, and/or a pharmaceutically acceptable carrier. Pharmaceutically acceptable carriers, which are useful, and include, but are not limited to, water, glycerol, saline, recombinant human albumin (e.g., RECOMBUMIN^{®}), ethanol, solubilized gelatins (e.g., GELOFUSINE^{®}), and other pharmaceutically acceptable salt solutions such as phosphates and salts of organic acids.

Formulations may be employed in admixtures with conventional excipients, *i.e.,* pharmaceutically acceptable organic or inorganic carrier substances suitable for oral, transdermal enteral, parenteral, inhalational, intravenous, nasal, subcutaneous, or any other suitable mode of administration, known to the art. The pharmaceutical preparations may be sterilized and if desired mixed with auxiliary agents, e.g., stabilizers, lubricants, preservatives, wetting agents, salts for influencing osmotic pressure buffers, emulsifiers, coloring, flavoring and/or fragrance-conferring substances and the like. They may also be combined where desired with other active agents, e.g., other analgesic, hypnotic agents or anxiolytics. As used herein, "additional ingredients" include, but are not limited to, one or more ingredients that may be used as a pharmaceutical carrier.

The pharmaceutical composition of this invention may comprise a preservative from about 0.005% to 2.0% by total weight of the pharmaceutical composition. The preservative is used to prevent spoilage in the case of exposure to contaminants in the environment. Examples of preservatives useful in accordance with the invention include but are not limited to those selected from the group consisting of imidurea, benzyl alcohol, parabens, sorbic acid and combinations thereof.

The dosage forms of pharmaceutical compositions of this invention, for example, but not limited to suspensions, solutions, tablets, capsules, emulsions, dispersions, syrups, granules, transdermal patches, gels, powders, creams, pastes, suppositories, or sprays.

Powdered and granular formulations of the pharmaceutical preparation of this invention may be prepared by using known methods. Such formulations may be administered directly to a subject, made into, for example, tablets, capsules, or an oily or aqueous suspension or solution by addition of an oily or aqueous vehicle thereto. Each of these formulations may further comprises one or more of dispersing or wetting agent, suspending agent, ionic and non-ionic surfactants, and preservative. Additional excipients, such as fillers and sweetening, flavoring, or coloring agents, may also be included in these formulations.

Suspensions may be prepared by using conventional methods to achieve suspension of the active ingredient in an aqueous or oily vehicle. Oily vehicles include, for example, oily esters, ethyl alcohol, almond oil, vegetable oils such as olive, arachis, coconut, or sesame oil, fractionated vegetable oils, and mineral oils such as liquid paraffin. Oily suspensions may further comprise a thickening agent. Aqueous vehicles include, for example, water, and isotonic saline. Liquid suspensions may further comprise one or more additional ingredients including, but not limited to, suspending agents, coloring agents, dispersing or wetting agents, buffers, emulsifying agents, salts, flavorings, demulcents, preservatives, and sweetening agents. Known suspending agents include, but are not limited to, sodium alginate, sorbitol syrup, polyvinylpyrrolidone, hydrogenated edible fats, gum tragacanth, gum acacia, and cellulose derivatives such as sodium carboxymethylcellulose, hydroxypropylmethyl cellulose, and methylcellulose. Known dispersing or wetting agents include, but are not limited to, naturally-occurring phosphatides such as lecithin, condensation products of an alkylene oxide with a fatty acid, with a partial ester derived from a fatty acid and a hexitol, with a long chain aliphatic alcohol, or with a partial ester derived from a fatty acid and a hexitol anhydride (e.g., polyoxyethylene sorbitol monooleate, polyoxyethylene stearate, polyoxyethylene sorbitan monooleate, and heptadecaethyleneoxycetanol, respectively). Known emulsifying agents include, but are not limited to, acacia, lecithin, and ionic or nonionic surfactants. Known preservatives include, but are not limited to, methyl, ethyl, or n-propyl para-hydroxybenzoates, sorbic acid, and ascorbic acid. Known sweetening agents include, for example, glycerol, sorbitol, propylene glycol, saccharin, and sucrose.

Liquid solutions of the active ingredient in aqueous or oily solvents may be prepared in substantially the same manner as liquid suspensions, with the primary difference being that the active ingredient is dissolved, rather than suspended in the solvent. As used herein, an "oily" liquid is one which comprises a carbon-containing liquid molecule and which exhibits a less polarity than that of water. Liquid solutions of the pharmaceutical composition of this invention may comprise each of the components described with regard to liquid suspensions. It should be understood that suspending agents will not necessarily aid dissolution of the active ingredient in the solvent. Oily solvents include, for example, oily esters, almond oil, ethyl alcohol, vegetable oils such as olive, arachis, coconut, or sesame oil, fractionated vegetable oils, and mineral oils such as liquid paraffin. Aqueous solvents include, for example, water, and isotonic saline.

A pharmaceutical composition of the invention may also be prepared, packaged, or sold in the form of water-in-oil emulsion or an oil-in-water emulsion. The oily phase may be a vegetable oil such as arachis or olive oil, a mineral oil such as liquid paraffin, or a combination of these. Such compositions may further comprise one or more emulsifying agents such as naturally occurring gums such as gum tragacanth or gum acacia, naturally-occurring phosphatides such as soybean or lecithin phosphatide, esters or partial esters derived from combinations of fatty acids and hexitol anhydrides such as sorbitan monooleate, and condensation products of such partial esters with ethylene oxide such as polyoxyethylene sorbitan monooleate. These emulsions may also contain additional ingredients including, for example, flavoring or sweetening agents.

Methods for impregnating or coating a material with a chemical composition are known in the art, and include, but are not limited to methods of binding or depositing a chemical composition onto a surface, methods of incorporating a chemical composition into the structure of a material during the synthesis of the material *(i.e.,* such as with a physiologically degradable material), and methods of absorbing an oily or aqueous solution or suspension into an absorbent material, with or without subsequent drying. Methods for mixing components include physical milling, the use of pellets in solid and suspension formulations and mixing in a transdermal patch, as known to those skilled in the art.

The terms "treat", "treating", "treatment" and "treatment method" as used herein, means reducing the frequency or severity with which symptoms of a disease or condition are experienced by a subject by virtue of administering an agent or pharmaceutical composition to the subject.

As used herein, the terms "patient", "individual" and "subject" may refer to a human or non-human mammal. Non-human mammals include, for example, livestock and pets, such as canine, feline, ovine, bovine, porcine and murine mammals. Preferably, the subject is human.

As used herein, the term "cancer" includes any type of malignant tumor, for example, but not limited to carcinoma or sarcoma. Cancer is caused by uncontrolled and/or abnormal proliferation of cells. And then the cells would invade and destroy the tissues around them. As used herein, the term "proliferation" refers to the cells after mitosis. As used herein, the term "metastasis" refers to the malignant tumor which is away from the original region. The cancer cells may metastasis by the blood stream, lymphatic system, passing through a body cavity, or the combination thereof.

The term "carcinoma" refers to the malignant epithelial cells which are new growth. These malignant epithelial cells preferred to invade the surrounded tissues and then metastasis.

The term "cancer vaccine" refers to a vaccine which would stimulate the immune system to against cancer or against the substances which facilitate cancer development. There are two types of cancer vaccines: (1) preventive cancer vaccine which is used to avoid the cancer development in healthy subjects; and (2) therapeutic cancer vaccine which is used to enhance the natural defense of the body to against cancer, so as to treat cancer (Lollini et al., Nature Reviews Cancer, 2006; 6(3):204-216). As used herein, the term "cancer vaccine" includes preventive cancer vaccine and therapeutic cancer vaccine.

Ranges: throughout this invention, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible sub-ranges as well as individual numerical values within that range. For example, description of a range such as from 1 to 5 should be considered to have specifically disclosed sub-ranges such as from 1 to 3, from 1 to 4, from 1 to 5, from 2 to 4, from 2 to 5, from 3 to 5 etc., as well as individual and partial numbers within that range, for example, 1, 2, 2.5, 3, 4, and 5. This applies regardless of the breadth of the range.

GMI used in this embodiment has the efficacy of inhibiting cancer cell growth. The major mechanism is that GMI could inhibit cancer cell growth induced by epidermal growth factor (EGF) through blocking the epidermal growth factor receptor (EGFR) signal transduction pathway. Therefore, GMI could further inhibit cancer cells metastasis and infiltration.

A pharmaceutical composition of this invention includes a ganoderma microsporum immunomodulatory protein (GMI), a phytohemagglutinin (PHA) and a keyhole limpet hemocyanin (KLH).

In this embodiment, the dose of GMI is from 5 µg/ml to 200 µg/ml. Preferably, the dose of GMI may be 5 µg/ml, 10 µg/ml, 15 µg/ml, 20 µg/ml, 25 µg/ml, 30 µg/ml, 35 µg/ml, 40 µg/ml, 45 µg/ml, 50 µg/ml, 55 µg/ml, 60 µg/ml, 65 µg/ml, 70 µg/ml, 75 µg/ml, 80 µg/ml, 85 µg/ml, 90 µg/ml, 95 µg/ml, 100 µg/ml, 105 µg/ml, 110 µg/ml, 115 µg/ml, 120 µg/ml, 125 µg/ml, 130 µg/ml, 135 µg/ml, 140 µg/ml, 145 µg/ml, 150 µg/ml, 155 µg/ml, 160 µg/ml, 165 µg/ml, 170 µg/ml, 175 µg/ml, 180 µg/ml, 185 µg/ml, 190 µg/ml, 195 µg/ml, or 200 µg/ml. Of course, the dose of GMI may be any value and range encompassed between any two values within the foregoing ranges and may be changed according to the carrier which is used, the route of administration, or the individual who in need and the physiology state thereof.

In this embodiment, the dose of KLH is from 10 µg/ml to 200 µg/ml. Preferably, the dose of KLH may be 10 µg/ml, 15 µg/ml, 20 µg/ml, 25 µg/ml, 30 µg/ml, 35 µg/ml, 40 µg/ml, 45 µg/ml, 50 µg/ml, 55 µg/ml, 60 µg/ml, 65 µg/ml, 70 µg/ml, 75 µg/ml, 80 µg/ml, 85 µg/ml, 90 µg/ml, 95 µg/ml, 100 µg/ml, 105 µg/ml, 110 µg/ml, 115 µg/ml, 120 µg/ml, 125 µg/ml, 130 µg/ml, 135 µg/ml, 140 µg/ml, 145 µg/ml, 150 µg/ml, 155 µg/ml, 160 µg/ml, 165 µg/ml, 170 µg/ml, 175 µg/ml, 180 µg/ml, 185 µg/ml, 190 µg/ml, 195 µg/ml, or 200 µg/ml. Of course, the dose of KLH may be any value and range encompassed between any two values within the foregoing ranges and may be changed according to the dose of GMI, the carrier which is used, the route of administration, or the individual who in need and the physiology state thereof.

In this embodiment, a weight ratio of GMI to KLH is from 20:1 to 1:40. Preferably, the weight ratio of GMI to KLH may be 20:1, 19:1, 18:1, 17:1, 16:1, 15:1, 14:1, 13:1, 12:1, 11:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:21, 1:22, 1:23, 1:24, 1:25, 1:26, 1:27, 1:28, 1:29, 1:30, 1:31, 1:32, 1:33, 1:34, 1:35, 1:36, 1:37, 1:38, 1:39, or 1:40. Of course, the weight ratio of GMI to KLH may be any value and range encompassed between any two ratios within the foregoing ranges and may be changed according to the carrier which is used, the route of administration, or the individual who in need and the physiology state thereof.

In this embodiment, the dose of PHA is from 1 µg/ml to 20 µg/ml. Preferably, the dose of PHA may be 1 µg/ml, 2 µg/ml, 3 µg/ml, 4 µg/ml, 5 µg/ml, 6 µg/ml, 7 µg/ml, 8 µg/ml, 9 µg/ml, 10 µg/ml, 11 µg/ml, 12 µg/ml, 13 µg/ml, 14 µg/ml, 15 µg/ml, 16 µg/ml, 17 µg/ml, 18 µg/ml, 19 µg/ml, or 20 µg/ml. Of course, the dose of PHA may be any value and range encompassed between any two values within the foregoing ranges and may be changed according to the dose of GMI, the carrier which is used, the route of administration, or the individual who in need and the physiology state thereof.

In this embodiment, a weight ratio of GMI to PHA is from 200:1 to 1:4. Preferably, the weight ratio of GMI to PHA may be 200:1, 195:1, 190:1, 185:1, 180:1, 175:1, 170:1, 165:1, 160:1, 155:1, 150:1, 145:1, 140:1, 135:1, 130:1, 125:1, 120:1, 115:1, 110:1, 105:1, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 4:1, 3:1, 2:1, 1:1, 1:2, 1:3, or 1:4. Of course, the weight ratio of GMI to PHA may be any value and range encompassed between any two ratios within the foregoing ranges and may be changed according to the carrier which is used, the route of administration, or the individual who in need and the physiology state thereof.

In this embodiment, a weight ratio of KLH to PHA is from 200:1 to 1:2. Preferably, the weight ratio of KLH to PHA may be 200:1, 195:1, 190:1, 185:1, 180:1, 175:1, 170:1, 165:1, 160:1, 155:1, 150:1, 145:1, 140:1, 135:1, 130:1, 125:1, 120:1, 115:1, 110:1, 105:1, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 4:1, 3:1, 2:1, 1:1, or 1:2. Of course, the weight ratio of KLH to PHA may be any value and range encompassed between any two ratios within the foregoing ranges and may be changed according to the carrier which is used, the route of administration, or the individual who in need and the physiology state thereof.

In this embodiment, the dosage form of the pharmaceutical composition is dispersion, suspension, solution, tablet, capsule, emulsion, syrup, granule, transdermal patch, gel, powder, cream, paste, suppository, liquid spray or the combination thereof. Preferably, the dosage form of the pharmaceutical composition may be dispersion, suspension or solution.

This invention also provides a pharmaceutical composition for use in the treatment of carcinoma, wherein the pharmaceutical composition includes GMI and KLH. In addition, also described is a method for treating carcinoma in a patient. The method includes providing a pharmaceutical composition comprising GMI and KLH. The concentration or dose of the pharmaceutical composition, the types of the carriers, the dosage forms of the pharmaceutical composition and other properties are mostly the same as those of the pharmaceutical composition described above, and therefore is omitted here for conciseness.

In this embodiment, the carcinoma is lung cancer, breast cancer, oral cancer, esophageal cancer, stomach cancer, colon cancer, hepatoma, cervical carcinoma, bladder cancer, pancreatic cancer, cutaneous carcinoma or the combination thereof. Preferably, the carcinoma is lung cancer, breast cancer, pancreatic cancer, bladder cancer or the combination thereof.

As mentioned above, the pharmaceutical composition of this invention, and the use and the described method thereof can achieve the efficacy of treating carcinoma.

To illustrate the efficacy of the use, the described method and the pharmaceutical composition of this invention for treating carcinoma, there are several examples shown below.

### Materials and methods

Cell lines: (1) A549 cells: lung cancer cell line (ATCC), the cell culture protocol please refer to Cooper JR. et al. PLoS One. 2016 Oct 28;11(10):e0164438. A549 cells were incubated in cell culture medium (RPMI-1640 containing 10% FBS) in a 37°C/5% CO₂ incubator. (2) MDA-MB-231 cells: Human breast cancer cell line (Bioresource Collection and Research Center, BCRC), the cell culture protocol please refer to Kim SW. et al. Int J Oncol. 2015 May;46(5):2067-75. MDA-MB-231 cells were incubated in cell culture medium (RPMI-1640 containing 10% FBS) in a 37°C/5% CO₂ incubator. (3) T24 cells: Bladder cancer cell line (BCRC), the cell culture protocol please refer to Zhao ZF. et al. Med Sci Monit. 2017 Mar 6;23:1156-1164. T24 cells were incubated in cell culture medium (RPMI-1640 containing 10% FBS) in a 37°C/5% CO₂ incubator. (4) BxPC-3 cells: Pancreatic cancer cell line (BCRC), the cell culture protocol please refer to Chen RY. et al. World J Gastroenterol. 2014 Oct 28; 20(40):14895-14903. BxPC-3 cells were incubated in cell culture medium (RPMI-1640 containing 10% FBS) in a 37°C/5% CO₂ incubator. Wherein, FBS was purchased from Biological Industries.

Human peripheral blood mononuclear cells (PBMCs): PBMCs were obtained from the whole blood samples of donors. The whole blood samples were centrifuged with Ficoll to isolate PBMCs. Then, PBMCs were cryopreserved with freezing medium. The frozen PBMCs could be thawed for the following experiments. PBMCs centrifuge protocol by using Ficoll and the cryopreserved protocol are commonly understood by one of ordinary skill in the art, and will be omitted here. Wherein, Ficoll was purchased from GE Healthcare.

Preparation of flow cytometry reagents: 7-AAD/CFSE Cell-Mediated Cytotoxicity Assay Kit (ImmunoChemistry Technologies LLC., Cat: #969) was used. 10X Assay Buffer was diluted to 1X Assay Buffer (4 mL of 10X Assay Buffer + 36 mL of sterilized water). CFSE was reconstituted with 200 µL of DMSO to form the CFSE stock. Then, CFSE stock was diluted by ratio of 1: 125 with 1X Assay Buffer to form 20X CFSE staining buffer (8 µL of CFSE stock + 992 µL of 1X Assay Buffer). 7-AAD was reconstituted with 260 µL of DMSO to form the 7-AAD stock. Then, 7-AAD stock was diluted by ratio of 1:10 with 1X Assay Buffer to form 21X 7-AAD staining buffer (40 µL of 7-AAD stock + 360 µL of 1X Assay Buffer).

Preparation of the treatment groups: (1) Control group: 1 mL of cell culture medium. (2) PHA groups (high/low dose): 1 mL of cell culture medium with 10 µg of PHA to form PHA 10 µg/mL group (low-dose PHA group); 1 mL of cell culture medium with 20 µg of PHA to form PHA 20 µg/mL group (high-dose PHA group). (3) KLH groups (high/low dose): 1 mL of cell culture medium with 100 µg of KLH to form KLH 100 µg/mL group (low-dose KLH group); 1 mL of cell culture medium with 200 µg of KLH to form KLH 200 µg/mL group (high-dose KLH group). (4) GMI groups (high/low dose): 1 mL of cell culture medium with 20 µg of GMI to form GMI 20 µg/mL group (low-dose GMI group); 1 mL of cell culture medium with 200 µg of GMI to form GMI 200 µg/mL group (high-dose GMI group). (5) pharmaceutical composition groups (high/low dose): 1 mL of cell culture medium with 10 µg of PHA, 100 µg of KLH and 20 µg of GMI to form PHA 10 µg/mL + KLH 100 µg/mL + GMI 20 µg/mL group (low-dose pharmaceutical composition group); 1 mL of cell culture medium with 20 µg of PHA, 200 µg of KLH and 200 µg of GMI to form PHA 20 µg/mL + KLH 200 µg/mL + GMI 200 µg/mL group (high-dose pharmaceutical composition group). Wherein, PHA was purchased from SIGMA-ALDRICH, Cat:L8902; KLH (Immucothel^{®}) was purchased from Biosyn.

### Example 1: the effects of GMI, KLH, PHA and pharmaceutical composition treatments on PBMCs

PBMCs (1 x 10⁶ cells/well) were suspended in (1) 1 mL of cell culture medium (control group); (2) 1 mL of cell culture medium with 10 µg/mL or 20 µg/mL of PHA (PHA group-1, PHA group-2); (3) 1 mL of cell culture medium with 100 µg/mL or 200 µg/mL of KLH (KLH group-1, KLH group-2); (4) 1 mL of cell culture medium with 20 µg/mL or 200 µg/mL of GMI (GMI group-1, GMI group-2); or (5) 1 mL of cell culture medium with PHA 10 µg/mL + KLH 100 µg/mL + GMI 20 µg/mL (pharmaceutical composition group-1) or 1 mL of cell culture medium with PHA 20 µg/mL + KLH 200 µg/mL + GMI 200 µg/mL (pharmaceutical composition group-2), respectively. Then, PBMCs were seeded in 24-well plates and were incubated for 24 hours in a 37°C/5% CO₂ incubator. After 24 hours incubation, PBMCs were observed with the optical microscope and took photos. Referring to FIG. 1A, the result of FIG. 1A shows that compared with the treatment of control group, high-dose or low-dose treatments of GMI groups, PHA groups and pharmaceutical composition groups would induce PBMCs aggregation, which means that the treatments of GMI groups, PHA groups and pharmaceutical composition groups would enhance the immune response of PBMCs.

After taking the photos, PBMCs were collected and transferred to micro-centrifuge tubes. Then, PBMCs were stained by 7-AAD staining buffer for 10 minutes. Next, PBMCs were analyzed with a flow cytometer (BD Accuri C6). The fluorescence density in PBMCs were identified by analyzing CFSE (FL-1 channel, green fluorescence) and 7-AAD (FL-3 channel, red fluorescence). The results of flow cytometer analysis were shown in FIG. 1B, the X-axis is the fluorescence density of CFSE, and the Y-axis is the fluorescence density of 7-AAD. Because PBMCs were merely stained by 7-AAD staining buffer, thus no CFSE fluorescence was detected. In FIG. 1B, the AAD quadrant presented live cells, and the AAD⁺ quadrant presented killed cells. Therefore, the formula for calculating the rate of cell cytotoxicity is (AAD⁺) / (AAD⁻ + AAD⁺) * 100%. The results in FIG. 1B were calculated by the formula of cell cytotoxicity to obtain the rates of cell cytotoxicity. And the results of rates of cell cytotoxicity from three independent experiments according to FIG. 1B were statistics in FIG. 1C.

Please refer to FIG. 1C, compared with the control group, merely high-dose and low-dose treatments of PHA group (marked as PHA group-1 and PHA group-2 in figures), low-dose GMI group (marked as GMI group-1 in figures) and low-dose pharmaceutical composition group (marked as pharmaceutical composition group-1 in figures) would cause a small part of PBMCs cytotoxicity. And the treatments of other groups have no effect on PBMCs cytotoxicity. According to the results of FIGs. 1A-1C, the treatments of PHA, KLH, GMI and pharmaceutical composition have almost no effect on the viability of PBMCs and would induce the immune response of PBMCs.

### Example 2: the effects of GMI, KLH, PHA and pharmaceutical composition treatments on A549 cells (lung cancer cells)

A549 cells were suspended in 19 mL of 1X Assay Buffer with a concentration of 1 x 10⁷ cells/mL. 1 mL of 20X CFSE staining buffer was added to A549 cells and incubated for 15 minutes at 37°C. Next, 20 mL of cell culture medium was added to A549 cells to terminate the binding reaction of CFSE. Then, A549 cells were centrifuged at 1200 rpm for 5 minutes. The supernatant was removed. A549 cell pellet was resuspended in 4 mL of cell culture medium, and seeded to 24-well plates (0.2 mL/well).

Next, A549 cells were distinguished into two populations. One is A549 treatment population (without PBMCs co-cultured). The other one is A549 + PBMC treatment population (A549 cells co-cultured with PBMCs to mimic the tumor environment in the body).

### A549 treatment population

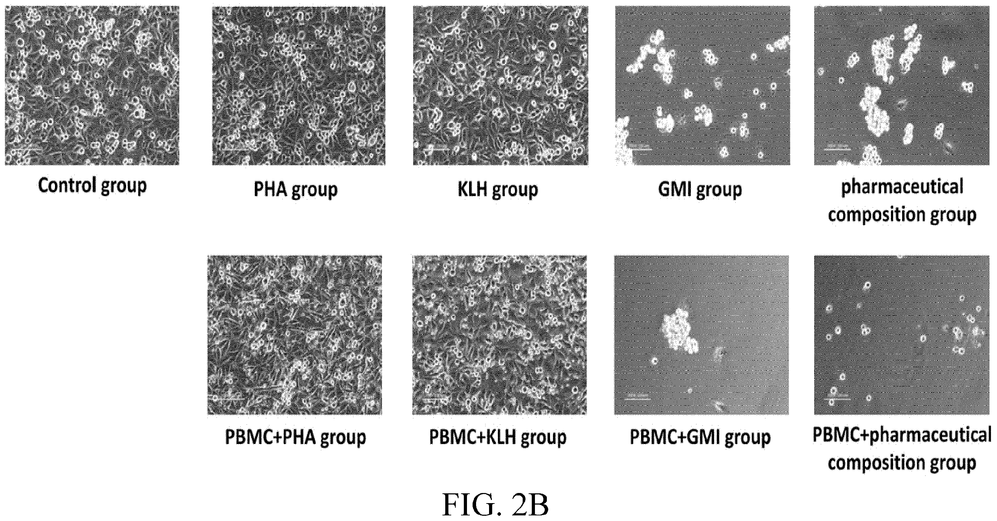

(1) 0.3 mL of cell culture medium (control group); (2) 0.3 mL of cell culture medium with PHA (PHA group); (3) 0.3 mL of cell culture medium with KLH (KLH group); (4) 0.3 mL of cell culture medium with GMI (GMI group); or (5) 0.3 mL of cell culture medium with PHA+KLH+GMI (pharmaceutical composition group) was added into A549 cells according to the treatment groups, respectively. Then, A549 cells were incubated for 24 hours in a 37°C/5% CO₂ incubator. After 24 hours incubation, the supernatants were collected and transferred to the micro-centrifuge tubes. 0.4 mL of DPBS was added into each well to wash A549 cells, and then the supernatant was collected and transferred to the micro-centrifuge tubes. 0.3 mL of DPBS was added to each well and A549 cells were observed with the optical microscope and took photos. Please refer to FIGs. 2A-2B, FIGs. 2A-2B show the cell photos of human lung cancer cell line (A549 cells) treated by PHA, KLH, GMI, or the pharmaceutical composition of this invention. The photos show the morphology of A549 cells which were observed with the optical microscope. FIG. 2A shows the result of low-dose treatment groups, and FIG. 2B shows the result of high-dose treatment groups. In FIG. 2A, PHA group has PHA 10 µg/mL; KLH group has KLH 100 µg/mL; GMI group has GMI 20 µg/mL; and pharmaceutical composition group has PHA 10 µg/mL + KLH 100 µg/mL + GMI 20 µg/mL. In FIG. 2B, PHA group has PHA 20 µg/mL; KLH group has KLH 200 µg/mL; GMI group has GMI 200 µg/mL; and pharmaceutical composition group has PHA 20 µg/mL + KLH 200 µg/mL + GMI 200 µg/mL. The results of FIGs. 2A-2B show that compared with the treatment of control group, high-dose or low-dose treatments of PHA group and KLH group would not change the numbers of the attached cells, which means that the treatments of PHA group and KLH group would not cause A549 cells cytotoxicity. However, compared with the treatment of control group, the treatments of GMI group and pharmaceutical composition group would decrease the numbers of the attached cells, which means that the treatments of GMI group and pharmaceutical composition group would cause A549 cells cytotoxicity.

After taking the photos, 0.3 mL of DPBS of each well was collected and transferred to the micro-centrifuge tube. 200 µL of Accutase^{™} cell detachment solution was added to each well to cover A549 cells. A549 cells were incubated at 37°C for 2 minutes until the cells were detached from the 24-well plates. The supernatants which were collected at the previous steps were added into each well and the supernatants with A549 cells were collected and transferred to micro-centrifuge tubes. Then A549 cells were stained by 7-AAD staining buffer for 10 minutes. Next, A549 cells were analyzed with a flow cytometer (BD Accuri C6). The fluorescence density in A549 cells were identified by analyzing CFSE (FL-1 channel, green fluorescence) and 7-AAD (FL-3 channel, red fluorescence). The results of flow cytometer analysis were shown in FIGs. 2C-2D, the X-axis is the fluorescence density of CFSE, and the Y-axis is the fluorescence density of 7-AAD. In FIGs. 2C-2D, the CFSE⁺AAD⁻ quadrant presented live cells, and the CFSE⁺AAD⁺ quadrant presented killed cells. Therefore, the formula for calculating the rate of cell cytotoxicity is (CFSE⁺AAD⁺) / (CFSE⁺AAD⁻ + CFSE⁺AAD⁺) * 100%. The results in FIGs. 2C-2D were calculated by the formula of cell cytotoxicity to obtain the rates of cell cytotoxicity. And the results of the rates of cell cytotoxicity from three independent experiments according to FIGs. 2C-2D were counted in FIGs. 2E-2F.

Please refer to FIGs. 2C-2F, in FIGs. 2C and 2E, PHA group has PHA 10 µg/mL; KLH group has KLH 100 µg/mL; GMI group has GMI 20 µg/mL; and pharmaceutical composition group has PHA 10 µg/mL + KLH 100 µg/mL + GMI 20 µg/mL. In FIGs. 2D and 2F, PHA group has PHA 20 µg/mL; KLH group has KLH 200 µg/mL; GMI group has GMI 200 µg/mL; and pharmaceutical composition group has PHA 20 µg/mL + KLH 200 µg/mL + GMI 200 µg/mL. The results of FIG. 2C which presented a single experiment analyzed by the flow cytometer shows that the rates of cell cytotoxicity of the control group, PHA group, KLH group, GMI group and pharmaceutical composition group are 0.9%, 4.6%, 1.0%, 8.1% and 17.4%, respectively. The results of FIG. 2E which presented the data of the rates of cell cytotoxicity statistics from three independent experiments analyzed by the flow cytometer under low-dose treatments show that PHA group treatment causes a small part of lung cancer cell cytotoxicity; KLH group treatment has no effect on lung cancer cell cytotoxicity; GMI group treatment causes higher lung cancer cell cytotoxicity than that of PHA group; and pharmaceutical composition group treatment causes higher lung cancer cell cytotoxicity than that of GMI group. This result shows that compared with PHA group, KLH group and GMI group treatments, pharmaceutical composition group (PHA+KLH+GMI) treatment has significantly higher cell cytotoxicity of lung cancer cells, which means pharmaceutical composition group treatment significantly caused lung cancer cells apoptosis, so as to has better treatment efficacy for lung cancer treatment. In addition, the results of FIG. 2D which presented a single experiment analyzed by the flow cytometer shows that the rates of cell cytotoxicity of the control group, PHA group, KLH group, GMI group and pharmaceutical composition group treatments are 0.9%, 13.8%, 1.4%, 29.3% and 39.5%, respectively. The results of FIG. 2F which presented the data of the rates of cell cytotoxicity statistics from three independent experiments analyzed by the flow cytometer under high-dose treatments show that PHA group treatment causes a small part of lung cancer cell cytotoxicity; KLH group treatment has no effect on lung cancer cell cytotoxicity; GMI group treatment causes higher lung cancer cell cytotoxicity than that of PHA group; and pharmaceutical composition group treatment causes higher lung cancer cell cytotoxicity than that of GMI group. This result shows that compared with PHA group, KLH group and GMI group treatments, pharmaceutical composition group (PHA+KLH+GMI) treatment has significantly higher cell cytotoxicity of lung cancer cells, which means pharmaceutical composition group treatment significantly caused lung cancer cells apoptosis, so as to has better treatment efficacy for lung cancer treatment. Compared with the result of low-dose treatments in FIG. 2E, the result of high-dose treatments in FIG. 2F shows that the apoptosis of lung cancer cells would increase significantly when the dose of pharmaceutical composition increased. As shown in FIGs. 2E-2F, "*" is *p* < 0.05 when compared between these two groups; "**" is *p* < 0.01 when compared between these two groups; "***" is *p* < 0.001 when compared between these two groups. All data are expressed as mean ± standard error of the mean (SEM) (n = 3); error bar presents SEM.

### A549 + PBMC treatment population

PBMCs (1 x 10⁷ cells) were suspended in 1 mL of cell culture medium and 0.1 mL of PBMCs were added in each well of 24-well plates (which contained 5 x 10⁵ A549 cells/well). In addition, (1) 0.2 mL of cell culture medium with PHA (PHA group); (2) 0.2 mL of cell culture medium with KLH (KLH group); (3) 0.2 mL of cell culture medium with GMI (GMI group); or (4) 0.2 mL of cell culture medium with PHA+KLH+GMI (pharmaceutical composition group) was added into each well of 24-well plates according to the treatment groups, respectively. Herein, the ratio of PBMCs and tumor cells is 2:1 to mimic the status of tumors in the human body. Then, the 24-well plates were incubated for 24 hours in a 37°C/5% CO₂ incubator. After 24 hours incubation, the supernatants were collected and transferred to the micro-centrifuge tubes. 0.4 mL of DPBS was added into each well to wash A549 cells, and then the supernatant was collected and transferred to the micro-centrifuge tubes. 0.3 mL of DPBS was added to each well and A549 cells were observed with the optical microscope and took photos. Please refer to FIGs. 2A-2B, the treatments with PBMCs were marked as PBMC+PHA group, PBMC+KLH group, PBMC+GMI group and PBMC+ pharmaceutical composition group. The results of FIGs. 2A-2B show that compared with the treatment of control group, high-dose or low-dose treatments of PBMC+PHA group and PBMC+KLH group would not change the numbers of the attached cells, which means that the treatments of PBMC+PHA group and PBMC+KLH group would not cause A549 cells cytotoxicity. However, compared with the treatment of control group, the treatments of PBMC+GMI group and PBMC + pharmaceutical composition group would decrease the numbers of the attached cells, which means that the treatments of PBMC+GMI group and PBMC + pharmaceutical composition group would cause A549 cells cytotoxicity.

After taking the photos, 0.3 mL of DPBS of each well was collected and transferred to the micro-centrifuge tube. 200 µL of Accutase^{™} cell detachment solution was added to each well to cover A549 cells. A549 cells were incubated at 37°C for 2 minutes until the cells were detached from the 24-well plates. The supernatants which were collected at the previous steps were added into each well and the supernatants with A549 cells were collected and transferred to micro-centrifuge tubes. Then A549 cells were stained by 7-AAD staining buffer for 10 minutes. Next, A549 cells were analyzed with a flow cytometer. The parameters of the flow cytometer, the protocol of flow cytometer analysis and the formula for calculating the rates of cell cytotoxicity were described in the previous paragraphs and will be omitted here.

Please refer to FIGs. 2C-2F, the treatments with PBMCs were marked as PBMC+PHA group, PBMC+KLH group, PBMC+GMI group and PBMC+ pharmaceutical composition group. The results of FIG. 2C which presented a single experiment analyzed by the flow cytometer under low-does treatments with PBMCs show that the rates of cell cytotoxicity of the control group, PBMC+PHA group, PBMC+KLH group, PBMC+GMI group and PBMC + pharmaceutical composition group treatments are 0.9%, 4.7%, 2.7%, 18.6% and 22.5%, respectively. The results of FIG. 2E which presented the data of the rates of cell cytotoxicity statistics from three independent experiments analyzed by the flow cytometer under low-dose treatments with PBMCs show that PHA group treatment causes a small part of lung cancer cell cytotoxicity; KLH group treatment has no effect on lung cancer cell cytotoxicity; GMI group treatment causes higher lung cancer cell cytotoxicity than that of PHA group; and pharmaceutical composition group treatment causes higher lung cancer cell cytotoxicity than that of GMI group. This result shows that compared with PHA group, KLH group and GMI group treatments, pharmaceutical composition group (PHA+KLH+GMI) treatment with PBMCs has significantly higher cell cytotoxicity of lung cancer cells, which means pharmaceutical composition group treatment significantly caused lung cancer cells apoptosis, so as to has better treatment efficacy for lung cancer treatment. In addition, the results of FIG. 2D which presented a single experiment analyzed by the flow cytometer under high-dose treatments with PBMCs show that the rates of cell cytotoxicity of the control group, PHA group, KLH group, GMI group and pharmaceutical composition group treatments are 0.9%, 13.9%, 4.7%, 48.6% and 55%, respectively. The results of FIG. 2F which presented the data of the rates of cell cytotoxicity statistics from three independent experiments analyzed by the flow cytometer under high-dose treatments with PBMCs show that PHA group treatment causes a small part of lung cancer cell cytotoxicity; KLH group treatment has no effect on lung cancer cell cytotoxicity; GMI group treatment causes higher lung cancer cell cytotoxicity than that of PHA group; and pharmaceutical composition group treatment causes higher lung cancer cell cytotoxicity than that of GMI group. This result shows that compared with PHA group, KLH group and GMI group treatments, pharmaceutical composition group (PHA+KLH+GMI) treatment with PBMCs has significantly higher cell cytotoxicity of lung cancer cells, which means pharmaceutical composition group treatment significantly caused lung cancer cells apoptosis, so as to has better treatment efficacy for lung cancer treatment. Compared with the result of low-dose treatment with PBMCs in FIG. 2E, the result of high-dose treatment with PBMCs in FIG. 2F shows that the apoptosis of lung cancer cells would increase significantly when the dose of pharmaceutical composition increased.

### The synergism of pharmaceutical composition of this invention for A546 cell cytotoxicity

There are three different effects of multiple drugs (more than two) have been proposed, including (1) synergism; (2) additive effect and (3) antagonism. The combination index (CI) published by Chou TC. et al. Quantitative analysis of dose-effect relationships: the combined effects of multiple drugs or enzyme inhibitors, Adv Enzyme Regul, Vol. 22, 1984, pp.27-55 and Liang Zhao et al. Evaluation of Combination Chemotherapy: Integration of Nonlinear Regression, Curve Shift, Isobologram, and Combination Index Analysis, Clinical Cancer Research, Vol. 10, 2004, pp.7994-8004 can be used in analyzing the drug-drug interaction. The calculation formula is CI = C_{A,x}/ IC_{x,A} + C_{B,x}/ IC_{x,B}. Wherein, C_{A,x} and C_{B,x} are the concentrations of drug A and drug B used in combination to achieve x% drug effect. IC_{x,A} and IC_{x,B} are the concentrations for single agents to achieve x% drug effect. CI < 1 indicates that the drugs used in combination have synergism. CI = 1 indicates that the drugs used in combination have additive effect. CI > 1 indicates that the drugs used in combination have antagonism.

In this example, the results of FIGs. 2E-2F which presented the data of the rates of cell cytotoxicity statistics from three independent experiments analyzed by the flow cytometer. As shown in FIG. 2E, the rates of cell cytotoxicity of the control group, PHA group (10 µg/mL), KLH group (100 µg/mL), GMI group (20 µg/mL), and pharmaceutical composition group (PHA 10 µg/mL + KLH 100 µg/mL + GMI 20 µg/mL) are 0.97%, 5.23%, 2.3%, 9.23% and 17.28%, respectively. As shown in FIG. 2F, the rates of cell cytotoxicity of the PHA group (20 µg/mL), KLH group (200 µg/mL), GMI group (200 µg/mL), and pharmaceutical composition group (PHA 20 µg/mL + KLH 200 µg/mL + GMI 200 µg/mL) are 11.83%, 1.7%, 23.83% and 40.53%, respectively. The cell cytotoxicity linear equations of PHA group, KLH group and GMI group were calculated by using these values. Take low-dose pharmaceutical composition group (PHA 10 µg/mL + KLH 100 µg/mL + GMI 20 µg/mL) treatment for example, the cell cytotoxicity 17.28% is put in y value of PHA cell cytotoxicity linear equation, KLH cell cytotoxicity linear equation, and GMI cell cytotoxicity linear equation, respectively, to obtain IC_{x,PHA}=27.62, IC_{x,KLH}=1557.42, and IC_{x,GMI}=117.05. Next, these values are put in the CI formula to obtain CI=(10/27.62)+(100/1557.42)+(20/117.05)=0.6. The CI value < 1 indicates that the pharmaceutical composition group has synergism on A549 cell cytotoxicity under low-dose treatment. The same method was used for calculating the CI value of the pharmaceutical composition group under high-dose treatment, and the value is 0.88. The CI value < 1 indicates that the pharmaceutical composition group also has synergism on A549 cell cytotoxicity under high-dose treatment. On the other hand, the CI value of the pharmaceutical composition group under low-dose treatment with PBMCs is 0.41; and the CI value of the pharmaceutical composition group under high-dose treatment with PBMCs is 0.96. The CI values are both < 1, and indicate that compared to PHA group, KLH group, and GMI group, the pharmaceutical composition group has synergism on A549 cell cytotoxicity under low/high-dose treatment with PBMCs.

### Example 3: the effects of GMI, KLH, PHA and pharmaceutical composition treatments on MDA-MB-231 cells (human breast cancer cells)

The experiment method of MDA-MB-231 cells in this example is similar to Example 2. The difference between Example 3 and Example 2 is merely used MDA-MB-231 cells instead of A549 cells to do the experiments. Therefore, the detailed experiment protocols will be omitted here.

### MDA-MB-231 treatment population

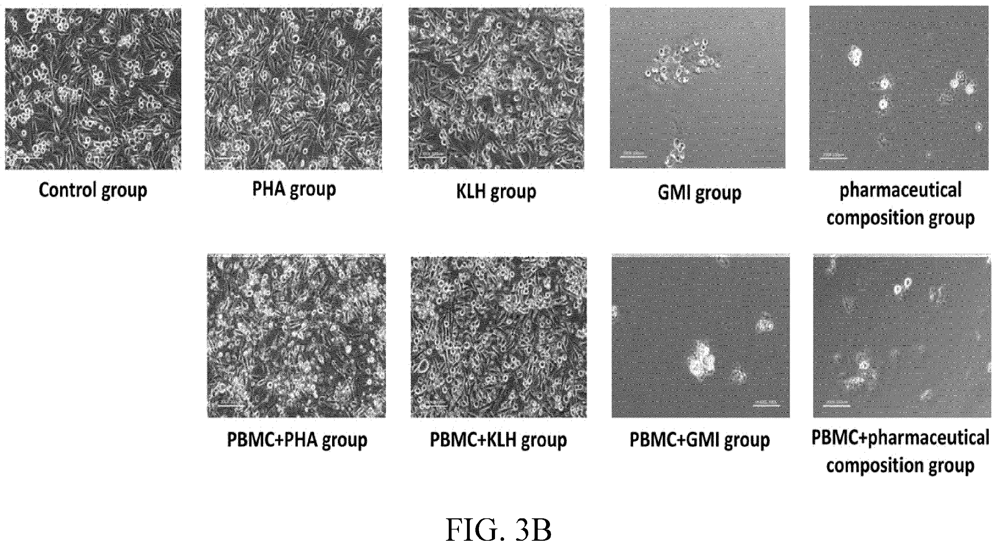

Please refer to FIGs. 3A-3B, FIGs. 3A-3B show the cell photos of human breast cancer cell line (MDA-MB-231 cells) treated by PHA, KLH, GMI, or the pharmaceutical composition of this invention. The photos show the morphology of MDA-MB-231 cells which were observed with the optical microscope. FIG. 3A shows the result of low-dose treatment groups, and FIG. 3B shows the results of high-dose treatment groups. In FIG. 3A, PHA group has PHA 10 µg/mL; KLH group has KLH 100 µg/mL; GMI group has GMI 20 µg/mL; and pharmaceutical composition group has PHA 10 µg/mL + KLH 100 µg/mL + GMI 20 µg/mL. In FIG. 3B, PHA group has PHA 20 µg/mL; KLH group has KLH 200 µg/mL; GMI group has GMI 200 µg/mL; and pharmaceutical composition group has PHA 20 µg/mL + KLH 200 µg/mL + GMI 200 µg/mL. The results of FIGs. 3A-3B show that compared with the treatment of control group, high-dose or low-dose treatments of PHA group and KLH group would not change the numbers of the attached cells, which means that the treatments of PHA group and KLH group would not cause MDA-MB-231 cells cytotoxicity. However, compared with the treatment of control group, the treatments of GMI group and pharmaceutical composition group would decrease the numbers of the attached cells, which means that the treatments of GMI group and pharmaceutical composition group would cause MDA-MB-231 cells cytotoxicity.

Please refer to FIGs. 3C-3F, in FIGs. 3C and 3E, PHA group has PHA 10 µg/mL; KLH group has KLH 100 µg/mL; GMI group has GMI 20 µg/mL; and pharmaceutical composition group has PHA 10 µg/mL + KLH 100 µg/mL + GMI 20 µg/mL. In FIGs. 3D and 3F, PHA group has PHA 20 µg/mL; KLH group has KLH 200 µg/mL; GMI group has GMI 200 µg/mL; and pharmaceutical composition group has PHA 20 µg/mL + KLH 200 µg/mL + GMI 200 µg/mL. The results of FIG. 3C which presented a single experiment analyzed by the flow cytometer show that the rates of cell cytotoxicity of the control group, PHA group, KLH group, GMI group and pharmaceutical composition group treatments are 1.8%, 5.4%, 2.8%, 44.7% and 54.5%, respectively. The results of FIG. 3E which presented the data of the rates of cell cytotoxicity statistics from three independent experiments analyzed by the flow cytometer under low-dose treatments show that PHA group and KLH group treatments have no effect on breast cancer cell cytotoxicity; GMI group treatment causes higher breast cancer cell cytotoxicity than those of PHA group and KLH group; and pharmaceutical composition group treatment causes higher breast cancer cell cytotoxicity than that of GMI group. This result shows that compared with PHA group, KLH group and GMI group treatments, pharmaceutical composition group (PHA+KLH+GMI) treatment has significantly higher cell cytotoxicity of breast cancer cells, which means pharmaceutical composition group treatment significantly caused breast cancer cells apoptosis, so as to has better treatment efficacy for breast cancer treatment. In addition, the results of FIG. 3D which presented a single experiment analyzed by the flow cytometer show that the rates of cell cytotoxicity of the control group, PHA group, KLH group, GMI group and pharmaceutical composition group treatments are 1.8%, 14.7%, 3.0%, 40.8% and 55.7%, respectively. The results of FIG. 3F which presented the data of the rates of cell cytotoxicity statistics from three independent experiments analyzed by the flow cytometer under high-dose treatments show that PHA group treatment causes a small part of breast cancer cell cytotoxicity; KLH group treatment has no effect on breast cancer cell cytotoxicity; GMI group treatment causes higher breast cancer cell cytotoxicity than that of PHA group; and pharmaceutical composition group treatment causes higher breast cancer cell cytotoxicity than that of GMI group. This result shows that compared with PHA group, KLH group and GMI group treatments, pharmaceutical composition group (PHA+KLH+GMI) treatment has significantly higher cell cytotoxicity of breast cancer cells, which means pharmaceutical composition group treatment significantly caused breast cancer cells apoptosis, so as to has better treatment efficacy for breast cancer treatment. Compared with the result of low-dose treatments in FIG. 3E, the result of high-dose treatments in FIG. 3F shows that the apoptosis of breast cancer cells would increase significantly when the dose of pharmaceutical composition increased. As shown in FIGs. 3E-3F, "*" is *p* < 0.05 when compared between these two groups; "***" is *p* < 0.001 when compared between these two groups. All data are expressed as mean ± SEM (n = 3); error bar presents SEM.

### MDA-MB-231 + PBMC treatment population

Please refer to FIGs. 3A-3B, the treatments with PBMCs were marked as PBMC+PHA group, PBMC+KLH group, PBMC+GMI group and PBMC+ pharmaceutical composition group. The results of FIGs. 3A-3B show that compared with the treatment of control group, high-dose or low-dose treatments of PBMC+PHA group and PBMC+KLH group would not change the numbers of the attached cells, which means that the treatments of PBMC+PHA group and PBMC+KLH group would not cause MDA-MB-231 cells cytotoxicity. However, compared with the treatment of control group, the treatments of PBMC+GMI group and PBMC + pharmaceutical composition group would decrease the numbers of the attached cells, which means that the treatments of PBMC+GMI group and PBMC + pharmaceutical composition group would cause MDA-MB-231 cells cytotoxicity.

Please refer to FIGs. 3C-3F, the treatments with PBMCs were marked as PBMC+PHA group, PBMC+KLH group, PBMC+GMI group and PBMC+ pharmaceutical composition group. The results of FIG. 3C which presented a single experiment analyzed by the flow cytometer under low-does treatments with PBMCs show that the rates of cell cytotoxicity of the control group, PBMC+PHA group, PBMC+KLH group, PBMC+GMI group and PBMC + pharmaceutical composition group treatments are 1.8%, 32.4%, 4.4%, 64.4% and 73.0%, respectively. The results of FIG. 3E which presented the data of the rates of cell cytotoxicity statistics from three independent experiments analyzed by the flow cytometer under low-dose treatments with PBMCs show that PHA group treatment causes a small part of breast cancer cell cytotoxicity; KLH group treatment has no effect on breast cancer cell cytotoxicity; GMI group treatment causes higher breast cancer cell cytotoxicity than that of PHA group; and pharmaceutical composition group treatment causes higher breast cancer cell cytotoxicity than that of GMI group. This result shows that compared with PHA group, KLH group and GMI group treatments, pharmaceutical composition group (PHA+KLH+GMI) treatment with PBMCs has significantly higher cell cytotoxicity of breast cancer cells, which means pharmaceutical composition group treatment significantly caused breast cancer cells apoptosis, so as to has better treatment efficacy for breast cancer treatment. In addition, the results of FIG. 3D which presented a single experiment analyzed by the flow cytometer under high-dose treatments with PBMCs show that the rates of cell cytotoxicity of the control group, PHA group, KLH group, GMI group and pharmaceutical composition group treatments are 1.8%, 48.5%, 4.3%, 59.0% and 64.7%, respectively. The results of FIG. 3F which presented the data of the rates of cell cytotoxicity statistics from three independent experiments analyzed by the flow cytometer under high-dose treatments with PBMCs show that PHA group treatment causes a small part of breast cancer cell cytotoxicity; KLH group treatment has no effect on breast cancer cell cytotoxicity; GMI group treatment causes higher breast cancer cell cytotoxicity than that of PHA group; and pharmaceutical composition group treatment causes higher breast cancer cell cytotoxicity than that of GMI group. This result shows that compared with PHA group, KLH group and GMI group treatments, pharmaceutical composition group (PHA+KLH+GMI) treatment with PBMCs has significantly higher cell cytotoxicity of breast cancer cells, which means pharmaceutical composition group treatment significantly caused breast cancer cells apoptosis, so as to has better treatment efficacy for breast cancer treatment.

### The synergism of pharmaceutical composition of this invention for MDA-MB-231 cell cytotoxicity

In this example, the CI value of pharmaceutical composition group under low-dose treatment without PBMCs is 0.21; and the CI value of pharmaceutical composition group under low-dose treatment with PBMCs is 0.45. The CI values are both < 1, and indicate that compared to PHA group, KLH group, and GMI group, the pharmaceutical composition group has synergism on MDA-MB-231 cell cytotoxicity under low-dose treatment with/without PBMCs.

### Example 4: the effects of GMI, KLH, PHA and pharmaceutical composition treatments on T24 cells (human bladder cancer cells)

The experiment method of T24 cells in this example is similar to Example 2. The difference between Example 4 and Example 2 is merely used T24 cells instead of A549 cells to do the experiments. Therefore, the detailed experiment protocols will be omitted here.

### T24 treatment population

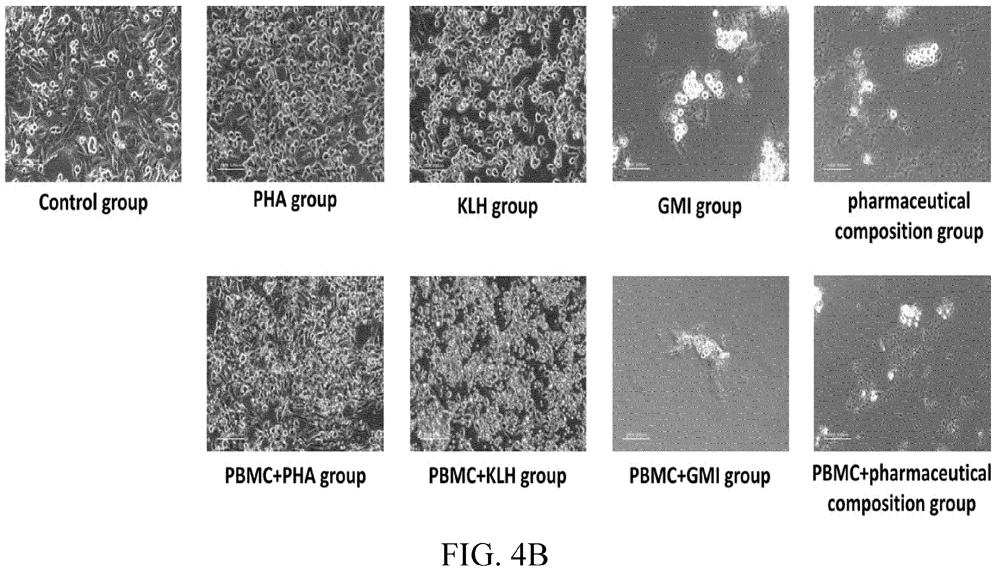

Please refer to FIGs. 4A-4B, FIGs. 4A-4B show the cell photos of human bladder cancer cell line (T24 cells) treated by PHA, KLH, GMI, or the pharmaceutical composition of this invention. The photos show the morphology of T24 cells which were observed with the optical microscope. FIG. 4A shows the result of low-dose treatment groups, and FIG. 4B shows the result of high-dose treatment groups. In FIG. 4A, PHA group has PHA 10 µg/mL; KLH group has KLH 100 µg/mL; GMI group has GMI 20 µg/mL; and pharmaceutical composition group has PHA 10 µg/mL + KLH 100 µg/mL + GMI 20 µg/mL. In FIG. 4B, PHA group has PHA 20 µg/mL; KLH group has KLH 200 µg/mL; GMI group has GMI 200 µg/mL; and pharmaceutical composition group has PHA 20 µg/mL + KLH 200 µg/mL + GMI 200 µg/mL. The results of FIGs. 4A-4B show that compared with the treatment of control group, high-dose or low-dose treatments of PHA group and KLH group would not change the numbers of the attached cells, which means that the treatments of PHA group and KLH group would not cause T24 cells cytotoxicity. However, compared with the treatment of control group, the treatments of GMI group and pharmaceutical composition group would decrease the numbers of the attached cells, which means that the treatments of GMI group and pharmaceutical composition group would cause T24 cells cytotoxicity.

Please refer to FIGs. 4C-4F, in FIGs. 4C and 4E, PHA group has PHA 10 µg/mL; KLH group has KLH 100 µg/mL; GMI group has GMI 20 µg/mL; and pharmaceutical composition group has PHA 10 µg/mL + KLH 100 µg/mL + GMI 20 µg/mL. In FIGs. 4D and 4F, PHA group has PHA 20 µg/mL; KLH group has KLH 200 µg/mL; GMI group has GMI 200 µg/mL; and pharmaceutical composition group has PHA 20 µg/mL + KLH 200 µg/mL + GMI 200 µg/mL. The results of FIG. 4C which presented a single experiment analyzed by the flow cytometer shows that the rates of cell cytotoxicity of the control group, PHA group, KLH group, GMI group and pharmaceutical composition group treatments are 3.1%, 22.1%, 2.8%, 50.2% and 66.9%, respectively. The results of FIG. 4E which presented the data of the rates of cell cytotoxicity statistics from three independent experiments analyzed by the flow cytometer under low-dose treatments show that PHA group treatment causes a small part of bladder cancer cell cytotoxicity; and KLH group treatment has no effect on bladder cancer cell cytotoxicity; GMI group treatment causes higher bladder cancer cell cytotoxicity than that of PHA group; and pharmaceutical composition group treatment causes higher bladder cancer cell cytotoxicity than that of GMI group. This result shows that compared with PHA group, KLH group and GMI group treatments, pharmaceutical composition group (PHA+KLH+GMI) treatment has significantly higher cell cytotoxicity of bladder cancer cells, which means pharmaceutical composition group treatment significantly caused bladder cancer cells apoptosis, so as to has better treatment efficacy for bladder cancer treatment. In addition, the results of FIG. 4D which presented a single experiment analyzed by the flow cytometer show that the rates of cell cytotoxicity of the control group, PHA group, KLH group, GMI group and pharmaceutical composition group treatments are 3.1%, 39.4%, 5.9%, 61.9% and 75.5%, respectively. The results of FIG. 4F which presented the data of the rates of cell cytotoxicity statistics from three independent experiments analyzed by the flow cytometer under high-dose treatments show, that PHA group treatment causes a small part of bladder cancer cell cytotoxicity; KLH group treatment has no effect on bladder cancer cell cytotoxicity; GMI group treatment causes higher bladder cancer cell cytotoxicity than that of PHA group; and pharmaceutical composition group treatment causes higher bladder cancer cell cytotoxicity than that of GMI group. This result shows that compared with PHA group, KLH group and GMI group treatments, pharmaceutical composition group (PHA+KLH+GMI) treatment has significantly higher cell cytotoxicity of bladder cancer cells, which means pharmaceutical composition group treatment significantly caused bladder cancer cells apoptosis, so as to has better treatment efficacy for bladder cancer treatment. Compared with the result of low-dose treatments in FIG. 4E, the result of high-dose treatments in FIG. 4F shows that the apoptosis of bladder cancer cells would increase significantly when the dose of pharmaceutical composition increased. As shown in FIGs. 4E-4F, "*" is *p* < 0.05 when compared between these two groups; "**" is *p* < 0.01 when compared between these two groups; "***" is *p* < 0.001 when compared between these two groups. All data are expressed as mean ± SEM (n = 3); error bar presents SEM.

### T24 + PBMC treatment population

Please refer to FIGs. 4A-4B, the treatments with PBMCs were marked as PBMC+PHA group, PBMC+KLH group, PBMC+GMI group and PBMC+ pharmaceutical composition group. The results of FIGs. 4A-4B show that compared with the treatment of control group, high-dose or low-dose treatments of PBMC+PHA group and PBMC+KLH group would not change the numbers of the attached cells, which means that the treatments of PBMC+PHA group and PBMC+KLH group would not cause T24 cells cytotoxicity. However, compared with the treatment of control group, the treatments of PBMC+GMI group and PBMC + pharmaceutical composition group would decrease the numbers of the attached cells, which means that the treatments of PBMC+GMI group and PBMC + pharmaceutical composition group would cause T24 cells cytotoxicity.

Please refer to FIGs. 4C-4F, the treatments with PBMCs were marked as PBMC+PHA group, PBMC+KLH group, PBMC+GMI group and PBMC+ pharmaceutical composition group. The results of FIG. 4C which presented a single experiment analyzed by the flow cytometer under low-does treatments with PBMCs show that the rates of cell cytotoxicity of the control group, PBMC+PHA group, PBMC+KLH group, PBMC+GMI group and PBMC + pharmaceutical composition group treatments are 3.1%, 28%, 5.0%, 62.3% and 68.5%, respectively. The results of FIG. 4E which presented the data of the rates of cell cytotoxicity statistics from three independent experiments analyzed by the flow cytometer under low-dose treatments with PBMCs show that PHA group treatment causes a small part of bladder cancer cell cytotoxicity; KLH group treatment has no effect on bladder cancer cell cytotoxicity; GMI group treatment causes higher bladder cancer cell cytotoxicity than that of PHA group; and pharmaceutical composition group treatment causes higher bladder cancer cell cytotoxicity than that of GMI group. This result shows that compared with PHA group, KLH group and GMI group treatments, pharmaceutical composition group (PHA+KLH+GMI) treatment with PBMCs has significantly higher cell cytotoxicity of bladder cancer cells, which means pharmaceutical composition group treatment with PBMCs significantly caused bladder cancer cells apoptosis, so as to has better treatment efficacy for bladder cancer treatment. In addition, the results of FIG. 4D which presented a single experiment analyzed by the flow cytometer under high-dose treatments with PBMCs show that the rates of cell cytotoxicity of the control group, PBMC+PHA group, PBMC+KLH group, PBMC+GMI group and PBMC + pharmaceutical composition group treatments are 3.1%, 40.9%, 6.9%, 80.8% and 84.0%, respectively. The results of FIG. 4F which presented the data of the rates of cell cytotoxicity statistics from three independent experiments analyzed by the flow cytometer under high-dose treatments with PBMCs show that PHA group treatment causes a small part of bladder cancer cell cytotoxicity; KLH group treatment has no effect on bladder cancer cell cytotoxicity; GMI group treatment causes higher bladder cancer cell cytotoxicity than that of PHA group; and pharmaceutical composition group causes higher bladder cancer cell cytotoxicity than that of GMI group. This result shows that compared with PHA group, KLH group and GMI group treatments, pharmaceutical composition group (PHA+KLH+GMI) treatment with PBMCs has significantly higher cell cytotoxicity of bladder cancer cells, which means pharmaceutical composition group treatment significantly caused bladder cancer cells apoptosis, so as to has better treatment efficacy for bladder cancer treatment. Compared with the result of low-dose treatments with PBMCs in FIG. 4E, the result of high-dose treatments with PBMCs in FIG. 4F shows that the apoptosis of bladder cancer cells would increase significantly when the dose of pharmaceutical composition increased.

### The synergism of pharmaceutical composition of this invention for T24 cell cytotoxicity

In this example, the CI value of pharmaceutical composition group under low-dose treatment without PBMCs is 0.53; and the CI value of pharmaceutical composition group under low-dose treatment with PBMCs is 0.56. The CI values are both < 1, and indicate that compared to PHA group, KLH group, and GMI group, the pharmaceutical composition group has synergism on T24 cell cytotoxicity under low-dose treatment with/without PBMCs.

### Example 5: the effects of GMI, KLH, PHA and pharmaceutical composition treatments on BxPC-3 cells (human pancreatic cancer cells)

The experiment method of BxPC-3 cells in this example is similar to Example 2. The difference between Example 5 and Example 2 is merely used BxPC-3 cells instead of A549 cells to do the experiments. Therefore, the detailed experiment protocols will be omitted here.

### BxPC-3 treatment population

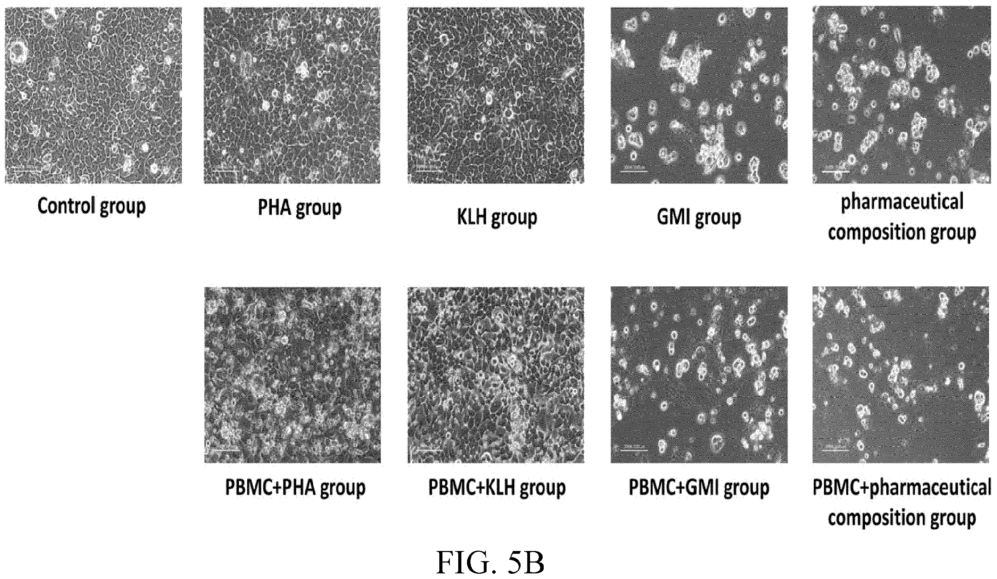

Please refer to FIGs. 5A-5B, FIGs. 5A-5B show the cell photos of human pancreatic cancer cell line (BxPC-3 cells) treated by PHA, KLH, GMI, or the pharmaceutical composition of this invention. The photos show the morphology of BxPC-3 cells which were observed with the optical microscope. FIG. 5A shows the results of low-dose treatment groups, and FIG. 5B shows the results of high-dose treatment groups. In FIG. 5A, PHA group has PHA 10 µg/mL; KLH group has KLH 100 µg/mL; GMI group has GMI 20 µg/mL; and pharmaceutical composition group has PHA 10 µg/mL + KLH 100 µg/mL + GMI 20 µg/mL. In FIG. 5B, PHA group has PHA 20 µg/mL; KLH group has KLH 200 µg/mL; GMI group has GMI 200 µg/mL; and pharmaceutical composition group has PHA 20 µg/mL + KLH 200 µg/mL + GMI 200 µg/mL. The results of FIGs. 5A-5B show that compared with the treatment of control group, high-dose or low-dose treatments of PHA group and KLH group would not change the numbers of the attached cells, which means that the treatments of PHA group and KLH group would not cause BxPC-3 cells cytotoxicity. However, compared with the treatment of control group, the treatments of GMI group and pharmaceutical composition group would decrease the numbers of the attached cells, which means that the treatments of GMI group and pharmaceutical composition group would cause BxPC-3 cells cytotoxicity.

Please refer to FIGs. 5C-5F, in FIGs. 5C and 5E, PHA group has PHA 10 µg/mL; KLH group has KLH 100 µg/mL; GMI group has GMI 20 µg/mL; and pharmaceutical composition group has PHA 10 µg/mL + KLH 100 µg/mL + GMI 20 µg/mL. In FIGs. 5D and 5F, PHA group has PHA 20 µg/mL; KLH group has KLH 200 µg/mL; GMI group has GMI 200 µg/mL; and pharmaceutical composition group has PHA 20 µg/mL + KLH 200 µg/mL + GMI 200 µg/mL. The results of FIG. 5C which presented a single experiment analyzed by the flow cytometer shows that the rates of cell cytotoxicity of the control group, PHA group, KLH group, GMI group and pharmaceutical composition group treatments are 7.0%, 7.9%, 5.2%, 14.3% and 25%, respectively. The results of FIG. 5E which presented the data of the rates of cell cytotoxicity statistics from three independent experiments analyzed by the flow cytometer under low-dose treatments show that PHA group and KLH group treatments have no effect on pancreatic cancer cell cytotoxicity; GMI group treatment causes higher pancreatic cancer cell cytotoxicity than that of PHA group and KLH group; and pharmaceutical composition group treatment causes higher pancreatic cancer cell cytotoxicity than that of GMI group. This result shows that compared with PHA group, KLH group and GMI group treatments, pharmaceutical composition group (PHA+KLH+GMI) treatment has significantly higher cell cytotoxicity of pancreatic cancer cells, which means pharmaceutical composition group treatment significantly caused pancreatic cancer cells apoptosis, so as to has better treatment efficacy for pancreatic cancer treatment. In addition, the results of FIG. 5D which presented a single experiment analyzed by the flow cytometer show that the rates of cell cytotoxicity of the control group, PHA group, KLH group, GMI group and pharmaceutical composition group treatments are 7.0%, 12.5%, 6.3%, 47.2% and 60.9%, respectively. The results of FIG. 5F which presented the data of the rates of cell cytotoxicity statistics from three independent experiments analyzed by the flow cytometer under high-dose treatments show that PHA group treatment causes a small part of pancreatic cancer cell cytotoxicity; KLH group treatment has no effect on pancreatic cancer cell cytotoxicity; GMI group treatment causes higher pancreatic cancer cell cytotoxicity than that of PHA group; and pharmaceutical composition group treatment causes higher pancreatic cancer cell cytotoxicity than that of GMI group. This result shows that compared with PHA group, KLH group and GMI group treatments, pharmaceutical composition group (PHA+KLH+GMI) treatment has significantly higher cell cytotoxicity of pancreatic cancer cells, which means pharmaceutical composition group treatment significantly caused pancreatic cancer cells apoptosis, so as to has better treatment efficacy for pancreatic cancer treatment. Compared with the result of low-dose treatments in FIG. 5E, the result of high-dose treatments in FIG. 5F shows that the apoptosis of pancreatic cancer cells would increase significantly when the dose of pharmaceutical composition increased. As shown in FIGs. 5E-5F, "*" is *p* < 0.05 when compared between these two groups; "**" is *p* < 0.01 when compared between these two groups; "***" is *p* < 0.001 when compared between these two groups. All data are expressed as mean ± SEM (n = 3); error bar presents SEM.

### BxPC-3 + PBMC treatment population

Please refer to FIGs. 5A-5B, the treatments with PBMCs were marked as PBMC+PHA group, PBMC+KLH group, PBMC+GMI group and PBMC+ pharmaceutical composition group. The results of FIGs. 5A-5B show that compared with the treatment of control group, high-dose or low-dose treatments of PBMC+PHA group and PBMC+KLH group would not change the numbers of the attached cells, which means that the treatments of PBMC+PHA group and PBMC+KLH group would not cause BxPC-3 cells cytotoxicity. However, compared with the treatment of control group, the treatments of PBMC+GMI group and PBMC + pharmaceutical composition group would decrease the numbers of the attached cells, which means that the treatments of PBMC+GMI group and PBMC + pharmaceutical composition group would cause BxPC-3 cells cytotoxicity.

Please refer to FIGs. 5C-5F, the treatments with PBMCs were marked as PBMC+PHA group, PBMC+KLH group, PBMC+GMI group and PBMC+ pharmaceutical composition group. The results of FIG. 5C which presented a single experiment analyzed by the flow cytometer under low-does treatments with PBMCs show that the rates of cell cytotoxicity of the control group, PBMC+PHA group, PBMC+KLH group, PBMC+GMI group and PBMC + pharmaceutical composition group treatments are 7.0%, 29.4%, 7.5%, 50.4% and 56.7%, respectively. The results of FIG. 5E which presented the data of the rates of cell cytotoxicity statistics from three independent experiments analyzed by the flow cytometer under low-dose treatments with PBMCs show that PHA group treatment causes a small part of pancreatic cancer cell cytotoxicity; KLH group treatment has no effect on pancreatic cancer cell cytotoxicity; GMI group treatment causes higher pancreatic cancer cell cytotoxicity than that of PHA group; and pharmaceutical composition group treatment causes higher pancreatic cancer cell cytotoxicity than GMI group. This result shows that compared with PHA group, KLH group and GMI group treatments, pharmaceutical composition group (PHA+KLH+GMI) treatment with PBMCs has significantly higher cell cytotoxicity of pancreatic cancer cells, which means pharmaceutical composition group treatment with PBMCs significantly caused pancreatic cancer cells apoptosis, so as to has better treatment efficacy for pancreatic cancer treatment. In addition, the results of FIG. 5D which presented a single experiment analyzed by the flow cytometer under high-dose treatments with PBMCs show that the rates of cell cytotoxicity of the control group, PBMC+PHA group, PBMC+KLH group, PBMC+GMI group and PBMC + pharmaceutical composition group treatments are 7.0%, 50%, 19.5%, 51.4% and 59.5%, respectively. The results of FIG. 5F which presented the data of the rates of cell cytotoxicity statistics from three independent experiments analyzed by the flow cytometer under high-dose treatments with PBMCs show that PHA group treatment causes a small part of pancreatic cancer cell cytotoxicity; KLH group treatment has no effect on pancreatic cancer cell cytotoxicity; GMI group treatment causes higher pancreatic cancer cell cytotoxicity than that of PHA group; and pharmaceutical composition group causes higher pancreatic cancer cell cytotoxicity than that of GMI group. This result shows that compared with PHA group, KLH group and GMI group treatments, pharmaceutical composition group (PHA+KLH+GMI) treatment with PBMCs has significantly higher cell cytotoxicity of pancreatic cancer cells, which means pharmaceutical composition group treatment significantly caused pancreatic cancer cells apoptosis, so as to has better treatment efficacy for pancreatic cancer treatment. Compared with the result of low-dose treatments with PBMCs in FIG. 5E, the result of high-dose treatments with PBMCs in FIG. 5F shows that the apoptosis of pancreatic cancer cells would increase significantly when the dose of pharmaceutical composition increased.

### The synergism of pharmaceutical composition of this invention for BxPC-3 cell cytotoxicity

In this example, the CI value of pharmaceutical composition group under low-dose treatment without PBMCs is 0.45; the CI value of pharmaceutical composition group under high-dose treatment without PBMCs is 0.9; and the CI value of pharmaceutical composition group under low-dose treatment with PBMCs is 0.52. The CI values are all < 1, and indicate that compared to PHA group, KLH group, and GMI group, the pharmaceutical composition group has synergism on BxPC-3 cell cytotoxicity under low-dose treatment with/without PBMCs and under high-dose treatment without PBMCs.

According to the result of Example 1, the GMI and PHA of the pharmaceutical composition of this invention would induce the immune response of immune cells (PBMCs) and have almost no effect on the viability of immune cells. According to the results of Example 2, compared with PHA group, KLH group and GMI group treatments, the treatment of the pharmaceutical composition of this invention may significantly cause human lung cancer cells (A549 cells) apoptosis and have synergism. In addition, when the dose of pharmaceutical composition increased, the apoptosis of lung cancer cells would increase significantly. Furthermore, the pharmaceutical composition of this invention would induce the immune response of immune cells so as to enhance cancer cell cytotoxicity of the pharmaceutical composition. According to the results of Example 3, compared with PHA group, KLH group and GMI group treatments, the treatment of the pharmaceutical composition of this invention may significantly cause human breast cancer cells (MDA-MB-231 cells) apoptosis and have synergism. In addition, when the dose of pharmaceutical composition increased, the apoptosis of breast cancer cells would increase significantly. Furthermore, the pharmaceutical composition of this invention would induce the immune response of immune cells so as to enhance cancer cell cytotoxicity of the pharmaceutical composition. According to the results of Example 4, compared with PHA group, KLH group and GMI group treatments, the treatment of the pharmaceutical composition of this invention may significantly cause human bladder cancer cells (T24 cells) apoptosis and have synergism. In addition, when the dose of pharmaceutical composition increased, the apoptosis of bladder cancer cells would increase significantly. Furthermore, the pharmaceutical composition of this invention would induce the immune response of immune cells so as to enhance cancer cell cytotoxicity of the pharmaceutical composition. According to the results of Example 5, compared with PHA group, KLH group and GMI group treatments, the treatment of the pharmaceutical composition of this invention may significantly cause pancreatic cancer cells (BxPC-3 cells) apoptosis and have synergism. In addition, when the dose of pharmaceutical composition increased, the apoptosis of pancreatic cancer cells would increase significantly. Furthermore, the pharmaceutical composition of this invention would induce the immune response of immune cells so as to enhance cancer cell cytotoxicity of the pharmaceutical composition. The examples are described for illustration but not intended to be limiting.

The previous cell culture experiment data could be used to prepare a dose range of pharmaceutical composition for human. Preferably, the dose range of pharmaceutical composition is within a range of circulating concentrations. The range of circulating concentration includes ED50 and has very low toxic or non-toxic. The effective dose of human treatment may be changed within the foregoing ranges according to the dosage form which is used and the route of administration. The effective dose of this invention for human treatment of the pharmaceutical composition may be estimated from the cell culture experiments data of this invention.

As mentioned above, the pharmaceutical composition and the use and the described method thereof of this invention can induce the specific active immune response of the immune cells against the tumor cells which can enhance cancer cell cytotoxicity of the pharmaceutical composition, so as to decrease the cancer cell viability to achieve the efficacy of tumor treatment.

## Claims

1. A pharmaceutical composition comprising a ganoderma microsporum immunomodulatory protein (GMI), a phytohemagglutinin (PHA) and a keyhole limpet hemocyanin (KLH).

2. The pharmaceutical composition of claim 1, wherein a concentration of GMI is from 5 µg/ml to 200 µg/ml.

3. The pharmaceutical composition of claim 1, wherein a concentration of KLH is from 10 µg/ml to 200 µg/ml.

4. The pharmaceutical composition of claim 1, wherein a weight ratio of GMI to KLH is from 20:1 to 1:40.

5. The pharmaceutical composition of claim 1, wherein a concentration of PHA is from 1 µg/ml to 20 µg/ml.

6. The pharmaceutical composition of claim 1, wherein a weight ratio of GMI to PHA is from 200:1 to 1:4, and a weight ratio of KLH to PHA is from 200:1 to 1:2.

7. The pharmaceutical composition of claim 1, wherein the pharmaceutical composition is in a dosage form selected from the group consisting of suspension, solution, tablet, capsule, emulsion, dispersion, syrup, granule, transdermal patch, gel, powder, cream, paste, suppository, liquid spray and the combination thereof.

8. A pharmaceutical composition for use in treatment of carcinoma, wherein the pharmaceutical composition comprises a ganoderma microsporum immunomodulatory protein (GMI), a phytohemagglutinin (PHA) and a keyhole limpet hemocyanin (KLH).

9. The pharmaceutical composition for use according to claim 8, wherein a concentration of GMI is from 5 µg/ml to 200 µg/ml.

10. The pharmaceutical composition for use according to claim 8, wherein a concentration of KLH is from 10 µg/ml to 200 µg/ml.

11. The pharmaceutical composition for use according to claim 8, wherein a weight ratio of GMI to KLH is from 20:1 to 1:40.

12. The pharmaceutical composition for use according to claim 8, wherein a concentration of PHA is from 1 µg/ml to 20 µg/ml.

13. The pharmaceutical composition for use according to claim 8, wherein a weight ratio of GMI to PHA is from 200:1 to 1:4, and a weight ratio of KLH to PHA is from 200:1 to 1:2.

14. The pharmaceutical composition for use according to claim 8, wherein the carcinoma is lung cancer, breast cancer, oral cancer, esophageal cancer, stomach cancer, colon cancer, hepatoma, cervical carcinoma, bladder cancer, pancreatic cancer, cutaneous carcinoma or the combination thereof.

## Patentansprüche

1. Pharmazeutische Zusammensetzung mit einem immunmodulatorischen Ganoderma-Mikrosporum-Protein (GMI), einem Phytohämagglutinin (PHA) und einem Schlitzschnecken-Hämocyanin (KLH).

2. Pharmazeutische Zusammensetzung gemäß Anspruch 1, wobei die Konzentration des GMI 5 µg/ml bis 200 µg/ml beträgt.

3. Pharmazeutische Zusammensetzung gemäß Anspruch 1, wobei die Konzentration des KLH 10 µg/ml bis 200 µg/ml beträgt.

4. Pharmazeutische Zusammensetzung gemäß Anspruch 1, wobei ein Gewichtsverhältnis von GMI zu KLH 20:1 bis 1:40 beträgt.

5. Pharmazeutische Zusammensetzung gemäß Anspruch 1, wobei eine Konzentration des PHA 1 µg/ml bis 20 µg/ml beträgt.

6. Pharmazeutische Zusammensetzung gemäß Anspruch 1, wobei ein Gewichtsverhältnis von GMI zu PHA 200:1 bis 1:4 beträgt und ein Gewichtsverhältnis von KLH zu PHA 200:1 bis 1:2 beträgt.

7. Pharmazeutische Zusammensetzung gemäß Anspruch 1, wobei die pharmazeutische Zusammensetzung in einer Darreichungsform vorliegt, die aus der Gruppe ausgewählt ist, die besteht aus Suspension, Lösung, Tablette, Kapsel, Emulsion, Dispersion, Sirup, Granulat, transdermalem Pflaster, Gel, Pulver, Creme, Paste, Zäpfchen, flüssigem Spray und der Kombination davon.

8. Pharmazeutische Zusammensetzung zur Verwendung bei der Behandlung von Karzinomen, wobei die pharmazeutische Zusammensetzung ein immunmodulatorisches Ganoderma-Mikrosporum-Protein (GMI), ein Phytohämagglutinin (PHA) und ein Schlitzschnecken-Hämocyanin (KLH) aufweist.

9. Pharmazeutische Zusammensetzung zur Verwendung gemäß Anspruch 8, wobei eine Konzentration des GMI 5 µg/ml bis 200 µg/ml beträgt.

10. Pharmazeutische Zusammensetzung zur Verwendung gemäß Anspruch 8, wobei eine Konzentration des KLH 10 µg/ml bis 200 µg/ml beträgt.

11. Pharmazeutische Zusammensetzung zur Verwendung gemäß Anspruch 8, wobei ein Gewichtsverhältnis von GMI zu KLH 20:1 bis 1:40 beträgt.

12. Pharmazeutische Zusammensetzung zur Verwendung gemäß Anspruch 8, wobei eine Konzentration des PHA 1 µg/ml bis 20 µg/ml beträgt.

13. Pharmazeutische Zusammensetzung zur Verwendung gemäß Anspruch 8, wobei ein Gewichtsverhältnis von GMI zu PHA 200:1 bis 1:4 beträgt und ein Gewichtsverhältnis von KLH zu PHA 200:1 bis 1:2 beträgt.

14. Pharmazeutische Zusammensetzung zur Verwendung gemäß Anspruch 8, wobei das Karzinom Lungenkrebs, Brustkrebs, Mundkrebs, Speiseröhrenkrebs, Magenkrebs, Dickdarmkrebs, Hepatom, Zervixkarzinom, Blasenkrebs, Bauchspeicheldrüsenkrebs, Hautkarzinom oder die Kombination davon ist.

## Revendications

1. Composition pharmaceutique comprenant une protéine immunomodulatoire de Ganoderma microsporum (GMI), une phytohémagglutinine (PHA) et une hémocyanine de patelle (KLH).

2. Composition pharmaceutique selon la revendication 1, dans laquelle une concentration en GMI est comprise entre 5 µg/ml et 200 µg/ml.

3. Composition pharmaceutique selon la revendication 1, dans laquelle une concentration en KLH est comprise entre 10 µg/ml et 200 µg/ml.

4. Composition pharmaceutique selon la revendication 1, dans laquelle un rapport pondéral entre la GMI et la KLH est compris entre 20:1 et 1:40.

5. Composition pharmaceutique selon la revendication 1, dans laquelle une concentration en PHA est comprise entre 1 µg/ml et 20 µg/ml.

6. Composition pharmaceutique selon la revendication 1, dans laquelle un rapport pondéral entre la GMI et la PHA est compris entre 200:1 et 1:4, et un rapport pondéral entre la KLH et la PHA est compris entre 200:1 et 1:2.

7. Composition pharmaceutique selon la revendication 1, dans laquelle la composition pharmaceutique est sous une forme posologique choisie dans le groupe constitué par une suspension, une solution, un comprimé, une gélule, une émulsion, une dispersion, un sirop, des granulés, un timbre transdermique, un gel, une poudre, une crème, une pâte, un suppositoire, un spray liquide et des combinaisons de ceux-ci.

8. Composition pharmaceutique pour une utilisation dans le traitement du carcinome, dans laquelle la composition pharmaceutique comprend une protéine immunomodulatoire de Ganoderma microsporum (GMI), une phytohémagglutinine (PHA) et une hémocyanine de patelle (KLH).

9. Composition pharmaceutique pour une utilisation selon la revendication 8, dans laquelle la concentration en GMI est comprise entre 5 µg/ml et 200 µg/ml.

10. Composition pharmaceutique pour une utilisation selon la revendication 8, dans laquelle la concentration en KLH est comprise entre 10 µg/ml et 200 µg/ml.

11. Composition pharmaceutique pour une utilisation selon la revendication 8, dans laquelle le rapport pondéral entre la GMI et la KLH est compris entre 20:1 et 1:40.

12. Composition pharmaceutique pour une utilisation selon la revendication 8, dans laquelle la concentration en PHA est comprise entre 1 µg/ml et 20 µg/ml.

13. Composition pharmaceutique pour une utilisation selon la revendication 8, dans laquelle le rapport pondéral entre la GMI et la PHA est compris entre 200:1 et 1:4, et le rapport pondéral entre la KLH et la PHA est compris entre 200:1 et 1:2.

14. Composition pharmaceutique pour une utilisation selon la revendication 8, dans laquelle le carcinome est un cancer du poumon, un cancer du sein, un cancer de la bouche, un cancer de l'œsophage, un cancer de l'estomac, un cancer du côlon, un hépatome, un carcinome cervical, un cancer de la vessie, un cancer du pancréas, un carcinome cutané ou une combinaison de ceux-ci.
